# EUROPÄISCHE PATENTSCHRIFT

(11) **EP 3 629 971 B1**
(45) Veröffentlichungstag und Bekanntmachung des Hinweises auf die Patenterteilung: **28.01.2026**
(21) Anmeldenummer: 18728526.7
(22) Anmeldetag: 14.05.2018
(51) Int. Cl.: A61B 34/00, A61B 1/00, A61B 34/30, B25J 1/02, A61B 90/50

(54) **ROBOTISCHER MANIPULATOR ZUR FÜHRUNG EINES ENDOSKOPS MIT PARALLELKINEMATIK**
ROBOTIC MANIPULATOR FOR GUIDING AN ENDOSCOPE, HAVING PARALLEL KINEMATICS
MANIPULATEUR ROBOTIQUE POUR GUIDER UN ENDOSCOPE AVEC UNE CINÉMATIQUE PARALLÈLE

(30) Priorität: 23.05.2017 DE 102017111296
(43) Veröffentlichungstag der Anmeldung: 08.04.2020
(73) Patentinhaber: Brainlab Robotics GmbH, 81829 München (DE)
(72) Erfinder: NOWATSCHIN, Stephan, 81677 München (DE); KRINNINGER, Maximilian, 82234 Wessling-Oberpfaffenhofen (DE); KÜHNAU, Christian, 81829 München (DE); ROPPENECKER, Daniel, 81829 München (DE); GIERLACH, Dominik, 80798 München (DE); AGRICOLA, Johannes, 81829 München (DE); WIGGER, Johann Ulrich, 81829 München (DE)
(74) Vertreter: Eisenführ Speiser
(86) Internationale Anmeldenummer: PCT/EP2018/062306
(87) Internationale Veröffentlichungsnummer: WO 2018/215221

(56) Entgegenhaltungen:
- EP-A1- 3 130 305
- WO-A2-01/34017
- DE-U1- 20 313 514

## Beschreibung

Die Erfindung betrifft eine chirurgische Manipulatorvorrichtung zum Positionieren eines chirurgischen Instruments, insbesondere Endoskops.

Chirurgische Manipulatorvorrichtungen sind grundsätzlich bekannt und werden in der Chirurgie insbesondere an einem Stativ oder Haltearm angebracht, um während einer Operation oder einer anderen Untersuchung bestimmte chirurgische Instrumente zu halten.

Eine solche chirurgische Manipulatorvorrichtung ist beispielsweise aus EP 1 658 016 B1 und aus WO 01/34017 A2 bekannt. Die dort offenbarte Vorrichtung ist dazu vorgesehen, ein Endoskop zu halten und ist grundsätzlich motorlos ausgebildet. Die Vorrichtung weist einen Rahmen auf, mit einer mindestens zwei Lenker umfassenden Lenkeranordnung, welche den Rahmen mit einem dem Instrument zugeordneten ersten Gelenkpunkt gelenkig verbindet, und mit einer mindestens zwei Lenker umfassenden zweiten Lenkeranordnung, welche den Rahmen mit einem dem ersten Instrument zugeordneten zweiten Gelenkpunkt gelenkig verbindet, wobei die Lenker der ersten Lenkeranordnung relativ zueinander und relativ zum Rahmen um Schwenkachsen schwenkbar gelagert sind und die Schwenkachsen parallel zueinander verlaufen und wobei die beiden Lenkeranordnungen so ausgebildet sind, das der erste Gelenkpunkt in einer ersten Bewegungsebene und der zweite Gelenkpunkt in einer zweiten Bewegungsebene bewegbar ist, dadurch gekennzeichnet, dass die erste Bewegungsebene relativ zur zweiten Bewegungsebene bewegbar ist. Dies wird bei EP 1 658 016 dazu genutzt, eine sich ändernde Distanz der ersten und zweiten Gelenkpunkte bei unterschiedlicher Verschwenkung bzw. Bewegung der ersten und der zweiten Lenkeranordnungen auszugleichen. Dies ist insbesondere deshalb erforderlich, da das Instrument direkt mit den Gelenkpunkten verbunden ist. Nachteilig an einem solchen Gerät ist insbesondere, dass die Anbindung des Rahmens an ein Stativ schwierig ist, da auch der Rahmen die Bewegung der Ebene erlauben muss. Weiterhin ist die Stabilität und Steifigkeit eines solchen Systems für manche Anwendungen in der Chirurgie nicht ausreichend.

Die chirurgische Manipulatorvorrichtung der vorliegenden Erfindung soll insbesondere an einem Haltearm aufgenommen werden, wie in DE 10 2014 016 823 A1, DE 10 2014 016 824 A1, DE 10 2015 104 810 A1, DE 10 2015 104 819 A1 und EP 3 130 305 A1 beschrieben.

Eine Aufgabe der vorliegenden Erfindung ist es daher, eine chirurgische Manipulatorvorrichtung der eingangs genannten Art bereitzustellen, die mit dem Haltearm, offenbart in DE 10 2014 016 823 A1, DE 10 2014 016 824 A1, DE 10 2015 104 810 A1, DE 10 2015 104 819 A1 und EP 3 130 305 A1, koppelbar ist, wobei der chirurgische Manipulator eine hohe Steifigkeit, einen kleinen Bauraum, eine hohe Positioniergenauigkeit sowie ein möglichst freies Arbeits- und Sichtfeld für den Chirurgen bereitstellt.

Diese Aufgabe wird bei einer chirurgischen Manipulatorvorrichtung der eingangs genannten Art gemäß Anspruch 1 dadurch gelöst, dass diese aufweist: einen Rahmen, einen ersten Halter und einen zweiten Halter zum Halten einer Instrumentenaufnahme für das chirurgische Instrument, eine erste an dem Rahmen gelagerte Lenkeranordnung, welche den Rahmen mit dem ersten Halter gelenkig verbindet, und eine zweite Lenkeranordnung, welche den Rahmen mit dem zweiten Halter gelenkig verbindet, wobei die ersten und zweiten Lenkeranordnung jeweils in zueinander parallelen und beabstandeten ersten und zweiten Bewegungsebenen relativ zum Rahmen bewegbar sind, sodass der erste Halter in der ersten Bewegungsebene und der zweite Halter in der zweiten Bewegungsebene bewegbar ist, wobei die erste Lenkeranordnung an vier Hebelgelenkpunkten der ersten Lenkeranordnung mit dem Rahmen gekoppelt ist, und die zweite Lenkeranordnung an vier Hebelgelenkpunkten der zweiten Lenkeranordnung mit dem Rahmen gekoppelt ist, dabei hat jeweils ein Hebelgelenkpunkt der ersten Lenkeranordnung eine gemeinsame Rotationsachse mit einem Hebelgelenkpunkt der zweiten Lenkeranordnung. Erfindungsgemäß ist damit eine Manipulatorvorrichtung geschaffen, die die ersten und zweiten Halter in separaten und stets parallel zueinander angeordneten Bewegungsebenen bewegen kann. Eine Verschwenkung der Ebenen zueinander, wie dies im Stand der Technik vorgeschlagen wird, wird von der vorliegenden chirurgischen Manipulatorvorrichtung nicht umgesetzt. Hierdurch kann ein Gelenk in dem Rahmen entfallen, und der Rahmen kann insgesamt steifer ausgebildet werden. Jede Lenkeranordnung ist zudem über vier Hebelgelenkpunkte mit dem Rahmen gekoppelt, wodurch wiederum eine höhere Steifigkeit erreicht wird. Zum reinen Positionieren der ersten und zweiten Halter reichen grundsätzlich zwei Hebelgelenkpunkte je Lenkeranordnung aus. Die jeweils zwei weiteren, die erfindungsgemäß vorgesehen sind, dienen dann insbesondere zur Stabilisierung.

Im Folgenden ist ein Verfahren zum Positionieren eines chirurgischen Instruments, ein Ankopplungselement und eine Instrumentenaufnahmeeinrichtung offenbart. Diese sind jedoch nicht beansprucht und dienen lediglich dem besseren Verständnis der Erfindung.

Vorzugsweise sind die vier ersten Hebelgelenkpunkte und die vier zweiten Hebelgelenkpunkte jeweils V-förmig angeordnet sind. Hierdurch wird weiterhin vermieden, dass die Lenkeranordnungen Singularitäten einnehmen können. Durch die V-förmige Anordnung der vier Hebelgelenkpunkte je Lenkeranordnung wird erreicht, dass jede Position der ersten und zweiten Halter eindeutig ist. Geometrisch oder statisch unbestimmte Positionen werden vermieden. Hierdurch ist insbesondere auch die Sicherheit der chirurgischen Manipulatorvorrichtung wesentlich erhöht, da es während einer Operation nicht zu Singularitäten in der Kinematik kommen kann. Zwar ist grundsätzlich auch eine Anordnung der vier ersten Hebelgelenkpunkte und der vier zweiten Hebelgelenkpunkte jeweils in einem Rechteck im Rahmen der Erfindung bevorzugt, allerdings sollten dann andere Mittel vorgesehen werden, um Singularitäten auszuschließen, wie etwas ein Begrenzen der Bewegungsfreiheit.

Der Winkel des V liegt vorzugsweise in einem Bereich von größer 0° bis einschließlich 90°. Es hat sich gezeigt, dass solche Winkel besonders bevorzugt sind. Bevorzugt sind ferner tendenziell kleinere Winkel, beispielsweise 45° oder weniger, 30° oder weniger, oder 20° oder weniger. Hierdurch wird die Manipulatorvorrichtung kompakter gestaltet.

Gemäß einer ersten bevorzugten Ausführungsform ist die zweite Lenkeranordnung im Wesentlichen spiegelsymmetrisch zu der ersten Lenkeranordnung ausgebildet. Die ersten und zweiten Lenkeranordnungen sind vorzugsweise in sich spiegelsymmetrisch, bezogen auf eine Ebene, die senkrecht auf die Bewegungsebenen steht. Die zweite Lenkeranordnung kann spiegelsymmetrisch zu der ersten Lenkeranordnung ausgebildet sein oder identisch zur ersten Lenkeranordnung und um einen Abstand versetzt. Hierdurch wird die Teileanzahl verringert, und die Anzahl an Gleichteilen kann erhöht werden. Hierdurch ist insbesondere der Fertigungsaufwand verringert.

ERfindungsgemäß sind die vier Hebelgelenkpunkte der ersten Lenkeranordnung und die vier Hebelgelenkpunkte der zweiten Lenkeranordnung auf gemeinsamen vier Rotationsachsen angeordnet. Das heißt, jeweils ein Gelenkpunkt der ersten Lenkeranordnung hat eine gemeinsame Rotationsachse mit einem Hebelgelenkpunkt der zweiten Lenkeranordnung. Beispielsweise hat der erste Hebelgelenkpunkt eine gemeinsame Rotationsachse mit dem fünften Hebelgelenkpunkt, der zweite Hebelgelenkpunkt hat eine gemeinsame Rotationsachse mit dem sechsten Hebelgelenkpunkt, der dritte Hebelgelenkpunkt hat eine gemeinsame Rotationsachse mit dem siebten Hebelgelenkpunkt, und der vierte Hebelgelenkpunkt hat eine gemeinsame Rotationsachse mit dem achten Hebelgelenkpunkt. Hierdurch wird die Konstruktion weiter vereinfacht, und die Lenkeranordnungen können symmetrisch ausgebildet werden. Hierdurch ist insbesondere die Bewegungsfreiheit und der Arbeitsraum der chirurgischen Manipulatorvorrichtung verbessert.

In einer Variante hierzu sind die vier Hebelgelenkpunkte der ersten Lenkeranordnung und die vier Hebelgelenkpunkte der zweiten Lenkeranordnung nicht auf gemeinsamen vier Rotationsachsen angeordnet. Die Rotationsachsen sind parallel versetzt zueinander. Hierdurch ist die Initiallage der Halter zueinander verschoben, wodurch sich ein initialer Anstellwinkel realisieren lässt. Dies hat den Vorteil, einen applikationsspezifischen bzw. patientenspezifischen Anstellwinkel des mittels der Halter aufnehmbaren chirurgischen Instruments zu realisieren. Dadurch kann beispielsweise die Zugänglichkeit für Instrumente zu einem Operationsgebiet verbessert werden und/oder der Arbeitsraum des Manipulators kann effizienter genutzt werden.

Gemäß einer weiteren bevorzugten Ausführungsform ist vorgesehen, dass die ersten und zweiten Lenkeranordnungen aus insgesamt genau drei verschiedenen Lenkerelementen gebildet sind. Diese drei verschiedenen Elemente sind vorzugsweise Hebel, Lenker und Stäbe. Diese drei verschiedenen Elemente werden im Folgenden noch näher erläutert werden. Die Erfinder haben erkannt, dass es ausreichend ist, diese drei verschiedenen Elemente einzusetzen, um die ersten und zweiten Lenkeranordnungen zu bilden. Hierdurch ist die Konstruktion einfach, und es kann auch Gleichteile zurückgegriffen werden. Hierdurch werden auch Kosten reduziert.

Vorzugsweise weist die erste Lenkeranordnung einen ersten, einen zweiten, einen dritten und einen vierten Hebel auf, die jeweils drehbar an ersten, zweiten, dritten und vierten Hebelgelenkpunkten der ersten Lenkeranordnung an dem Rahmen drehbar gelagert sind. Ferner weist die erste Lenkeranordnung einen ersten Lenker auf, der mit den ersten und zweiten Hebeln drehbar gekoppelt ist. Sie weist ferner einen zweiten Lenker auf, der mit den dritten und vierten Hebeln drehbar gekoppelt ist. Vorzugsweise definieren die Rotationsachsen der ersten und zweiten Hebel einen ersten Schenkel des V und die Rotationsachsen des dritten und vierten Hebels einen zweiten Schenkel des V.

Weiterhin ist vorgesehen, dass die erste Lenkeranordnung erste und zweite Stäbe aufweist, die einerseits drehbar mit dem ersten Lenker und andererseits drehbar mit dem ersten Halter gekoppelt sind. Entsprechend weist die erste Lenkeranordnung dritte und vierte Stäbe auf, die einerseits drehbar mit dem zweiten Lenker und andererseits drehbar mit dem ersten Halter gekoppelt sind. So ist der erste Halter über die erste Lenkeranordnung an dem Rahmen gekoppelt.

Die ersten und zweiten Stäbe sind vorzugsweise parallel zueinander angeordnet. Die dritten und vierten Stäbe sind ebenfalls vorzugsweise parallel zueinander angeordnet.

In entsprechender Weise gilt für die zweite Lenkeranordnung, dass diese einen fünften, einen sechsten, einen siebten und einen achten Hebel aufweist, die jeweils drehbar an fünften, sechsten, siebten und achten Hebelgelenkpunkten der zweiten Lenkeranordnung an dem Rahmen drehbar gelagert sind. Die zweite Lenkeranordnung weist einen dritten Lenker auf, der mit den fünften und sechsten Hebeln drehbar gekoppelt ist und einen vierten Lenker, der mit den siebten und achten Hebeln drehbar gekoppelt ist. Weiterhin ist vorgesehen, dass die zweite Lenkeranordnung fünfte und sechste Stäbe aufweist, die einerseits drehbar mit dem dritten Lenker und andererseits drehbar mit dem zweiten Halter gekoppelt sind. In entsprechender Weise weist die zweite Lenkeranordnung siebte und achte Stäbe auf, die einerseits drehbar mit dem vierten Lenker und andererseits drehbar mit dem zweiten Halter gekoppelt sind. Auf diese Weise ist der zweite Halter, wie dies auch schon mit Bezug auf den ersten Halter beschrieben wurde, mit dem Rahmen gekoppelt.

Vorzugsweise sind die fünften und sechsten Stäbe parallel zueinander angeordnet. Ebenso ist bevorzugt, dass die siebten und achten Stäbe parallel zueinander angeordnet sind.

Gemäß einer weiteren bevorzugten Ausführungsform weist die erste Lenkeranordnung ein erstes, ein zweites, ein drittes und ein viertes Parallelogramm auf. Vorzugsweise weist auch die zweite Lenkeranordnung ein fünftes, ein sechstes, ein siebtes und ein achtes Parallelogramm auf.

Vorzugsweise wird das erste Parallelogramm von den ersten und zweiten Hebeln, dem ersten Lenker und dem Rahmen gebildet. Das zweite Parallelogramm wird vorzugsweise aus den ersten und zweiten Stäben, dem ersten Lenker und dem ersten Halter gebildet.

In entsprechender Weise wird vorzugsweise das dritte Parallelogramm von den dritten und vierten Hebeln, dem zweiten Lenker und dem Rahmen gebildet. Das vierte Parallelogramm wird vorzugsweise von den dritten und vierten Stäben, dem zweiten Lenker und dem ersten Halter gebildet. Die dritten und vierten Parallelogramme sind insbesondere zur Stabilisierung vorgesehen; die ersten und zweiten Parallelogramme zur Positionierung des ersten Halters. In gleicher Weise ist vorzugsweise das fünfte Parallelogramm aus dem fünften und sechsten Hebel, dem dritten Lenker und dem Rahmen gebildet. Das sechste Parallelogramm wird vorzugsweise aus den fünften und sechsten Stäben, dem dritten Lenker und dem zweiten Halter gebildet. Das siebte Parallelogramm wird vorzugsweise aus den siebten und achten Hebeln, dem vierten Lenker und dem Rahmen gebildet. Das achte Parallelogramm wird entsprechend vorzugsweise aus den siebten und achten Stäben, dem vierten Lenker und dem zweiten Halter gebildet. Auch das sechste und achte Parallelogramm dient insbesondere zur Stabilisierung, während das fünfte und siebte Parallelogramm zur Positionierung dient.

Durch die Hintereinanderschaltung der Parallelogramme wird erreicht, dass der erste Halter nur in einer Ebene, nämlich erster Bewegungsebene in x- und y-Richtung bewegbar ist. Entsprechend ist der zweite Halter nur in der zweiten Bewegungsebene, auch dort in x- und y-Richtung bewegbar.

Weiterhin ist bevorzugt, dass das erste und zweite Parallelogramm einen gemeinsamen Gelenkpunkt aufweisen. Vorzugsweise weisen auch das dritte und vierte Parallelogramm einen gemeinsamen Gelenkpunkt auf. Ebenso weisen vorzugsweise das fünfte und sechste bzw. das siebte und achte Parallelogramm je einen gemeinsamen Gelenkpunkt auf. Hierdurch wird die Konstruktion weiter vereinfacht und die Baugröße der chirurgischen Manipulatorvorrichtung verkleinert. Da die Parallelogramme jeweils hintereinandergeschaltet sind, bilden die Lenker jeweils ein Element von zwei Parallelogrammen, sodass es möglich ist, gemeinsame Gelenkpunkte umzusetzen.

Gemäß einer weiteren bevorzugten Ausführungsform weist die chirurgische Manipulatorvorrichtung einen Antrieb für die ersten und zweiten Lenkeranordnungen auf. Hierdurch ist es möglich, die Lenkeranordnungen anzutreiben, um so die ersten und zweiten Halter in den ersten und zweiten Bewegungsebenen zu positionieren.

In einer bevorzugten Weiterbildung weist der Antrieb einen ersten und einen zweiten Motor für die erste Lenkeranordnung und einen dritten und einen vierten Motor für die zweite Lenkeranordnung auf. Die ersten, zweiten, dritten und vierten Motoren sind vorzugsweise unabhängig voneinander betreibbar. Sie sind vorzugsweise als Elektromotoren mit einer rotierenden Abtriebswelle ausgebildet. Vorzugsweise sind alle vier Motoren baugleich.

Vorzugsweise treiben der erste und zweite Motor die distal zum ersten Halter angeordneten Hebel an und der dritte und vierte Motor die proximal zum zweiten Halter angeordneten Hebel. Das heißt, vorzugsweise treibt der erste Motor den ersten Hebel an, der zweite Motor den dritten Hebel, der dritte Motor den sechsten Hebel und der vierte Motor den achten Hebel. Wenn die sich Hebel insgesamt immer paarweise eine Rotationsachse teilen, ist eine Anordnung erreicht, bei der jeweils von einem sich eine Rotationsachse teilenden Paar an Hebeln, jeweils ein Hebel angetrieben und der andere Hebel passiv ist. Hierdurch wird die Baugröße der chirurgischen Manipulatorvorrichtung deutlich reduziert. Die vier Motoren des Antriebs können so angeordnet sein, dass ihre Rotationsachsen jeweils parallel zueinander sind. Es ist nicht erforderlich, die einzelnen Motoren in etwa koaxial und axial zueinander versetzt anzuordnen, da sich keine zwei Motoren eine gemeinsame Rotationsachse teilen.

In einer bevorzugten Variante oder zusätzlich weist die chirurgische Manipulatorvorrichtung eine Bremseinrichtung zum aktiven Bremsen der ersten und zweiten Lenkeranordnungen sowie eine Freigabeeinheit zum selektiven Freigeben eines oder mehrerer Freiheitsgrade der ersten und/oder zweiten Lenkeranordnung auf. Die Bremseinrichtung weist vorzugsweise eine erste und eine zweite Bremse für die erste Lenkeranordnung, und eine dritte und eine vierte Bremse für die zweite Lenkeranordnung auf. Bevorzugt bremsen die erste und zweite Bremse die distal zum ersten Halter angeordneten Hebel, und die dritte und vierte Bremse bremsen die proximal zum zweiten Halter angeordneten Hebel. Insofern sind die Bremsen vorzugsweise analog zu den beschriebenen Motoren angeordnet und können anstatt der Motoren in dem Rahmen eingesetzt werden. Hierdurch ist eine passive chirurgische Manipulatorvorrichtung geschaffen, deren Bremseinrichtung insbesondere manuell mittels der Freigabeeinheit freigebbar ist, um so die Pose der der ersten und zweiten Lenkeranordnungen und die Position der ersten und zweiten Halter in den ersten und zweiten Bewegungsebenen zu verstellen.

Die Bremsen der Bremseinrichtung sind vorzugsweise im stromlosen Zustand zugespannt. Sie sind vorzugsweise als elektromagnetische Bremsen ausgebildet. Durch Bestromung der Bremsen werden diese gelöst, und die Pose der ersten und zweiten Lenkeranordnungen ist verstellbar.

Die Freigabeeinheit weist vorzugsweise einen Schalter oder dergleichen auf und ist mit der Bremseinrichtung gekoppelt. Die Bremseinrichtung kann über eine Bremssteuereinrichtung verfügen, die die Bremsen separat oder gemeinsam steuert. Die Freigabeeinheit ist vorzugsweise so ausgebildet, dass jede Bremse separat freigebbar und sperrbar ist und/oder alle Bremsen gemeinsam gelöst werden können. Die Freigabeeinheit ist vorzugsweise entfernt von dem Rahmen, der Bremseinrichtung und/oder den ersten und zweiten Lenkeranordnungen anordenbar, insbesondere an einem aufzunehmenden chirurgischen Instrument, auf einem Fußboden oder einem OP-Tisch, und kabelgebunden oder drahtlos mit der Bremseinrichtung zum Bereitstellen eines Freigabesignals verbunden. Die Freigabeeinheit kann beispielsweise als ein Fußpedal ausgebildet sein. In einer anderen Variante weist die Freigabeeinheit einen Schalter auf und einen Grundkörper und ist an ein chirurgisches Instrument, das an den ersten und zweiten Haltern aufgenommen wird, anklippbar. So ist es möglich, dass ein Bediener, wenn er das chirurgische Instrument ergreift, die Freigabeeinheit so betätigt, dass die Bremsen freigegeben werden und die Pose der ersten und zweiten Lenkeranordnungen, und so auch die Postiion des chirurgischen Instruments, manuell verstellbar ist. Hierdurch ist eine intuitive Bedienung geschaffen. Zusätzlich oder alternativ ist die Freigabeeinheit drahtlos mit der Bremseinrichtung bzw. einer Bremssteuereinrichtung der Bremseinrichtung verbunden, beispielsweise über WiFi, Bluetooth oder ein drahtloses Übertragungssystem einer höheren Nachfolgeordnung. Beispielsweise umfasst die Freigabeeinheit ein Softwareprogramm, das auf einem handgehaltenen Computer, beispielsweise einem Mobiltelefon, einem Tablet-PC oder dergleichen, ausgeführt wird. In einer weiteren Variante ist die Freigabeeinheit nach Art einer Fernbedienung gebildet und stellt das Freigabesignal mittels Infrarotstrahlung an der Bremseinrichtung bereit, die zu diesem Zweck mit einem entsprechenden Empfänger ausgestattet ist. Es ist aber auch denkbar, dass die Freigabeeinheit zusätzlich oder alternativ einen manuell betätigbaren Schalter aufweist, der an dem Gehäuse der chirurgischen Manipulatorvorrichtung angeordnet ist.

In einer weiteren bevorzugten Ausführungsform weist die chirurgische Manipulatorvorrichtung eine Instrumentenaufnahmeeinrichtung auf, die gelenkig mit dem ersten und dem zweiten Halter gekoppelt ist. Die Instrumentenaufnahmeeinrichtung weist vorzugsweise Formschlussmittel auf, die dazu eingerichtet sind, ein Ankopplungselement für das chirurgische Instrument aufzunehmen. Das chirurgische Instrument ist gemäß der Erfindung vorzugsweise nicht direkt mit den ersten und zweiten Haltern gekoppelt, sondern eine Instrumentenaufnahmeeinrichtung ist mit den ersten und zweiten Haltern gekoppelt, und das Instrument kann dann wahlweise mit der Instrumentenaufnahmeeinrichtung verbunden werden. Einerseits ist es denkbar, dass das chirurgische Instrument direkt mit der Instrumentenaufnahmeeinrichtung verbunden wird, bevorzugt ist aber die Verbindung des Instruments mit einem Ankopplungselement, das dann seinerseits formschlüssig mit der Instrumentenaufnahmeeinrichtung verbunden wird.

Die Instrumentenaufnahmeeinrichtung ist vorzugsweise so ausgelegt, dass sie einen sich ändernden Abstand zwischen dem ersten und zweiten Halter, aufgrund von verschiedenen Positionierungen der ersten und zweiten Halter in der ersten und zweiten Bewegungsebene zulässt. Beispielsweise ist es denkbar, dass die Instrumentenaufnahmeeinrichtung einerseits fest mit dem ersten Halter gekoppelt ist, während an dem zweiten Halter eine Gleitführung und/oder Rutschkupplung vorgesehen ist und die Instrumentenaufnahmeeinrichtung in dieser Gleitführung bzw. mittels der Rutschkupplung am zweiten Halter gehalten wird. Die Gleitführung ist vorzugsweise so ausgelegt, dass die Instrumentenaufnahmeeinrichtung relativ zum zweiten Halter beweglich ist. Hierdurch ist ein Ausgleich des sich ändernden Abstands zwischen erstem und zweitem Halter möglich, ohne die ersten und zweiten Bewegungsebenen gegeneinander zu verschwenken und ohne, dass die ersten und zweiten Halter die jeweiligen ersten und zweiten Bewegungsebenen verlassen müssen. Hierdurch werden Verspannungen vermieden. Darüberhinaus wird hierdurch die Torsionssteifigkeit an der Instrumentenaufnahmeeinrichtung erhöht und gleichzeitig die Steifigkeit in Instrumentenrichtung reduziert, was hinsichtlich der Patientensicherheit vorteilhaft ist.

Vorzugsweise ist das Ankopplungselement aus einem Isolationsmaterial gebildet, um, falls ein Instrument in dem Ankopplungselement aufgenommen ist, dieses gegenüber der Instrumentenaufnahmeeinrichtung elektrisch zu isolieren. Hierdurch ist die Sicherheit der chirurgischen Manipulatorvorrichtung wesentlich verbessert.

In einer weiteren bevorzugten Ausführungsform weist die Instrumentenaufnahmeeinrichtung einen Linearantrieb auf, zum Positionieren des Instruments wenigstens teilweise senkrecht zu den ersten und zweiten Bewegungsebenen. Hierdurch ist ein weiterer Freiheitsgrad für die chirurgische Manipulatorvorrichtung geschaffen, und das Instrument kann insbesondere senkrecht zu den Bewegungsebenen bewegt werden. Dies ist insbesondere dann vorteilhaft, wenn als Instrument beispielsweise ein Endoskop, ein Katheter oder eine Biopsienadel aufgenommen ist, also Instrumente, die im Wesentlichen auch entlang ihrer eigenen Längsachse während einer Untersuchung bewegt werden sollen.

In einer bevorzugten Weiterbildung weist der Linearantrieb eine längliche Hülse, einen in der Hülse angeordneten Spindeltrieb und ein Abtriebselement auf, das die Formschlussmittel trägt, wobei der Spindeltrieb einen in der Hülse angeordneten Magnetmitnehmer antreibt und das Abtriebselement äußerlich und linear verschieblich an der Hülse gelagert ist und mittels magnetischer Kraft mit dem Mitnehmer gekoppelt ist. Auf diese Weise ist es möglich, dass die Hülse äußerlich eben ist und keine Durchlässe, Nuten oder sonstigen Ausnehmungen aufweist. Hierdurch ist insbesondere die Hygiene verbessert. Der Magnetmitnehmer im Inneren der Hülse ist mit einem Spindeltrieb gekoppelt, und der Spindeltrieb treibt den Magnetmitnehmer an, um diesen linear zu bewegen. Ein Spindeltrieb hat allgemein den Vorteil, dass eine hohe Positioniergenauigkeit erreichbar ist, ohne zu hohe elektromagnetische Felder einzubringen, wie dies beispielsweise bei herkömmlichen elektromagnetischen Linearantrieben der Fall ist. Zum Antreiben der Spindel reicht ein klein dimensionierter Elektromotor aus, der an einem Ende der Hülse angeordnet sein kann. Aufgrund der rein magnetischen Kopplung zwischen dem Abtriebselement und dem Magnetmitnehmer ist es auch möglich, das Instrument samt Abtriebselement von der Instrumentenaufnahmeeinrichtung zu entnehmen, ohne die Instrumentenaufnahmeeinrichtung vollständig zu demontieren.

Gemäß einer weiteren bevorzugten Ausführungsform weist die Instrumentenaufnahmeeinrichtung einen Drehantrieb auf, der dazu vorgesehen ist, ein aufgenommenes chirurgisches Instrument um eine Drehachse zu drehen, wobei die Drehachse vorzugsweise im Wesentlichen parallel zu einer Antriebsrichtung des Linearantriebs verläuft. Hierdurch wird eine Drehung des chirurgischen Instruments um eine weitere Achse erlaubt. Üblicherweise wird ein chirurgisches Instrument so an der Instrumentenaufnahme aufgenommen, dass es entlang seiner Längsachse linear antreibbar ist, insbesondere in Bezug auf ein Endoskop, das entlang seiner Stabachse bewegbar ist. Für gewinkelte Optiken ist es bevorzugt, den Drehantrieb der vorliegenden Ausführungsform umzusetzen, um das Sichtfeld zu vergrößern.

Gemäß einer weiteren bevorzugten Ausführungsform ist vorgesehen, dass die Instrumentenaufnahmeeinrichtung eine Kraft-Momenten-Sensoreinheit aufweist, die dazu eingerichtet ist, Kräfte und Momente zu erfassen, die von einem an der Instrumentenaufnahmeeinrichtung aufgenommenen chirurgischen Instrument auf die Instrumentenaufnahmeeinrichtung wirken. Vorzugsweise ist die Kraft-Momenten-Sensoreinheit mit der Steuereinheit der chirurgischen Manipulatorvorrichtung gekoppelt, und die Steuereinheit weist Softwaremittel auf, die dazu eingerichtet sind, die von der Kraft-Momenten-Sensoreinheit bereitgestellten Signale zu verarbeiten und einen Antrieb der chirurgischen Manipulatoreinheit und/oder den Linearantrieb der Instrumentenaufnahmeeinrichtung oder einen anderen Antrieb der Instrumentenaufnahmeeinrichtung entsprechend zu steuern. Die Kraft, die von dem Instrument auf die Instrumentenaufnahmeeinrichtung wirkt, kann einen Bedienerwunsch darstellen. So ist es beispielsweise möglich, dass ein Operateur das Instrument, welches an der Instrumentenaufnahmeeinrichtung aufgenommen ist, manuell ergreift und manuell zu einem bestimmten Punkt führen will. In diesem Fall wirken von dem Instrument auf die Instrumentenaufnahmeeinrichtung Kräfte und Momente, die dann durch die Kraft-Momenten-Sensoreinheit erfasst werden. Das Steuern umfasst in dieser Variante vorzugsweise Ermitteln einer Bewegung und/oder Pose für die ersten und zweiten Lenkeranordnungen und/oder den Linearantrieb, um die auf die Instrumentenaufnahmeeinrichtung wirkenden Kräfte und Momente auszugleichen; sowie Steuern des Antriebs und/oder des Linearantriebs in Übereinstimmung mit dieser Bewegung oder Pose, um diese Bewegung und/oder Pose auszuführen bzw. einzunehmen.

Für den Fall, dass anstatt eines Bedieners die Kraft auf das aufgenommene Instrument durch einen Patienten ausgeübt wird, beispielsweise weil eine Fehlbedienung vorlag und die Manipulatorvorrichtung eine suboptimale Pose eingenommen hat oder sich der Patient bewegt hat, wird durch dieses Vorgehen auch diese Kraft verringert und Entlastung geschaffen. Die Sicherheit ist verbessert.

Weiterhin ist bevorzugt, dass die Steuereinheit Signale von der Kraft-Momenten-Sensoreinheit empfängt, die das Gewicht einer an der Instrumentenaufnahme aufgenommene Masse repräsentieren. Dieses Ladegewicht führt bei der chirurgischen Manipulatorvorrichtung zu einer leichten elastischen Verformung aufgrund der endlichen Steifigkeit des Systems. In der Steuereinheit der chirurgischen Manipulatorvorrichtung sind in diesem Ausführungsbeispiel Daten hinterlegt, die die Systemesteifigkeit repräsentieren. Bei der Bestimmung der Pose, um eine gewünschte Sollposition mit dem chirurgischen Instrument zu erreichen, wird dann vorzugsweise die Masse, die an der Instrumentenaufnahme aufgenommen ist, berücksichtigt. Zur Bestimmung der Pose werden in diesem Fall vorzugsweise folgende Schritte ausgeführt: Ermitteln einer Auslenkung aufgrund der aufgenommenen Masse in der Zielpose der ersten und zweiten Lenkeranordnungen, Ermitteln einer angepassten Zielpose basierend auf der ermittelten Auslenkung; und Bewegen der ersten und zweiten Halter mittels der ersten und zweiten Lenkeranordnungen in die angepasste Zielpose zum Positionieren des chirurgischen Instruments.

Gemäß einer weiteren bevorzugten Ausführungsform weist die chirurgische Manipulatorvorrichtung eine elektronische Steuereinheit mit Speichermitteln und einem Prozessor auf, zum Steuern einer Bewegung und Positionierung wenigstens des ersten und zweiten Halters. Die elektronische Steuereinheit ist vorzugsweise auch dazu eingerichtet den Linearantrieb der Instrumentenaufnahmeeinrichtung oder einen anderen Antrieb der Instrumentenaufnahmeeinrichtung zu steuern.

Weiterhin ist bevorzugt, dass die chirurgische Manipulatorvorrichtung eine elektronische Schnittstelle zum Empfang von Stellsignalen von einer übergeordneten Steuereinheit, insbesondere einem OP-Navigationssystem oder einem chirurgischen Haltearm mit der übergeordneten Steuereinheit, aufweist. Einerseits ist es denkbar, dass die chirurgische Manipulatorvorrichtung über die elektronische Schnittstelle direkt Stellsignale für den Antrieb der ersten und zweiten Lenkeranordnung empfängt und/oder den Linearantrieb der Instrumentenaufnahmeeinrichtung oder einen anderen Antrieb der Instrumentenaufnahmeeinrichtung und insoweit keine eigene Intelligenz benötigt. Andererseits ist es auch denkbar, dass die chirurgische Manipulatorvorrichtung die elektronische Steuereinheit mit Speichermitteln und Prozessor aufweist und insofern autonom in der Lage ist, Stellsignale für den Antrieb und/oder den Linearantrieb der Instrumentenaufnahmeeinrichtung oder einen anderen Antrieb der Instrumentenaufnahmeeinrichtung zu ermitteln, insbesondere basierend auf Eingaben eines Bedieners oder basierend auf Stellsignalen, die über die elektronische Schnittstelle empfangen werden. So ist es etwa denkbar, dass über die elektronische Schnittstelle eine gewünschte Sollposition des chirurgischen Instruments empfangen wird und die elektronische Steuereinheit der chirurgischen Manipulatorvorrichtung dann aus dieser Sollposition entsprechende Stellsignale für den Antrieb und/oder den Linearantrieb der Instrumentenaufnahmeeinrichtung oder einen anderen Antrieb der Instrumentenaufnahmeeinrichtung ermittelt, um den Antrieb und/oder den Linearantrieb der Instrumentenaufnahmeeinrichtung oder einen anderen Antrieb der Instrumentenaufnahmeeinrichtung entsprechend zu steuern zum Veranlassen der ersten und zweiten Lenkeranordnungen den ersten und zweiten Halter zu bewegen und zu positionieren und/oder den Linearantrieb der Instrumentenaufnahmeeinrichtung oder einen anderen Antrieb der Instrumentenaufnahmeeinrichtung entsprechend zu steuern. Die elektronische Schnittstelle kann in einer Variante als kabelgebundene Schnittstelle mit physischen Kontakten oder als Funkschnittstelle ausgebildet sein, die die Stellsignale von der übergeordneten Steuereinheit drahtlos empfängt. Hierzu sind insbesondere Wi-Fi-Schnittstellen, Bluetooth-Schnittstellen, Infrarotschnittstellen oder Nachfolgeschnittstellen einer höheren Entwicklungsstufe denkbar. Besonders bevorzug ist, dass die chirurgische Manipulatorvorrichtung mit einem Haltearm, wie eingangs beschrieben, gekoppelt ist und die Stellsignale von dem Haltearm empfangen werden. Der Haltearm kann in einer Ausführungsform diese Stellsignale selbst ermitteln, oder die Stellsignale werden von dem Haltearm von einem übergeordneten Steuersystem, beispielsweise einem OP-Navigationssystem, empfangen und an der chirurgische Manipulatorvorrichtung bereitgestellt.

Weiterhin ist bevorzugt, dass die chirurgische Manipulatorvorrichtung ein integriertes Eingabesystem zum Empfangen von Benutzereingaben aufweist. Das integrierte Eingabesystem ist vorzugsweise mit der Steuereinheit der Manipulatorvorrichtung gekoppelt, oder die chirurgische Manipulatorvorrichtung weist eine separate Steuereinheit für das integrierte Eingabesystem auf. Das integrierte Eingabesystem umfasst in einer Variante ein Mikrofon, um Sprachkommandos als Benutzereingaben zu empfangen. Die Steuereinheit, mit der das integrierte Eingabesystem vorzugsweise verbunden ist, weist in dieser Variante vorzugsweise wenigstens einen Prozessor und Softwaremittel auf, die dazu geeignet sind, Sprachkommandos zu verarbeiten und entsprechende Stellsignale an dem Antrieb der ersten und zweiten Lenkeranordnungen, dem Linearantrieb der Instrumentenaufnahmeeinrichtung, einem anderen Antrieb der Instrumentenaufnahmeeinrichtung und/oder der Bremseinrichtung bereitzustellen. So kann beispielsweise vorgesehen sein, dass bei Empfang eines Kommandos "freigeben" ein entsprechendes Freigabesignal von der Steuereinheit, die mit dem integrierten Eingabesystem verbunden ist, an der Bremseinrichtung bereitgestellt wird. In einer weiteren Variante werden Sprachkommandos des Benutzers lediglich aufgezeichnet und gespeichert, um diese dann in einem OP-Protokoll ausgeben zu können. Auf diese Weise ist es möglich, bestimmte Operationshandlungen anhand von Stellungen eines chirurgischen Instruments bestimmten Benutzereingaben zuzuordnen.

Weiterhin ist bevorzugt, dass das integrierte Eingabesystem wenigstens eine Kamera aufweist, die die ersten und zweiten Lenkeranordnungen, die Position der ersten und zweiten Halter, eine Position eines chirurgischen Instruments, das an den ersten und zweiten Halter aufgenommen ist, und/oder ein OP-Feld beobachtet. Über eine solche Kamera kann beispielsweise erfasst werden, welche Instrumente in einen OP-Bereich eingebracht und herausgeführt werden. Die Steuereinheit weist in dieser Ausführungsform vorzugsweise Softwaremittel auf, die dazu ausgebildet sind, anhand von Bilderkennungsalgorithmen in den OP-Bereich hereingeführte und herausgeführte Instrumente zu erkennen, entsprechende Signale mit einem Zeitstempel zu versehen und für ein OP-Protokoll zu speichern und bereitzustellen.

Gemäß einer weiteren bevorzugten Ausführungsform weist die chirurgische Manipulatorvorrichtung ein Gehäuse mit einer Anzeigeeinrichtung zum Anzeigen von einem oder mehreren Zuständen der Manipulatorvorrichtung auf. Derartige Zustände können insbesondere umfassen einen oder mehrere der folgenden: Bewegung der ersten und/oder zweiten Lenkeranordnungen, Bewegung eines Linearantriebs einer Instrumentenaufnahme, Empfang von Signalen, Senden von Signalen, Erreichen einer Sollposition der ersten und zweiten Halter, Erreichen und/oder Überschreiten eines vordefinierten Arbeitsraums der chirurgischen Manipulatorvorrichtung, Richtung, in die der erste und/oder zweite Halter bewegt werden bzw. zu bewegen sind, Art des aufgenommenen chirurgischen Instruments, Kraft, die auf das chirurgische Instrument wirkt, die chirurgische Manipulatorvorrichtung eingeschaltet ist, sich in einem Standby-Modus befindet, auf eine Benutzereingabe wartet, und/oder ein Softwareupdate empfängt, Erreichen oder Anfahren einer Homeposition der ersten und zweiten Lenkeranordnungen, sich die ersten und zweiten Lenkeranordnungen in oder nahe einer Extremposition befinden, den Zustand einer Kopplung mit einem Haltearm, den Zustand einer Verbindung eines Peripheriegeräts mit der elektronischen Schnittstelle, die Art des aufgenommenen Instruments, insbesondere 0°-Endoskop, 30°-Endoskop, 45°-Endoskop, Exoskop, schaltbare Winkeloptik, oder spezifische Geräte von Drittfirmen, das Abspeichern einer eingenommenen Pose der ersten und zweiten Lenkeranordnungen in einem internen oder externen Speicher, den Erfolg des Speicherns, eine graphische Darstellung der abgespeicherten Daten (z.B. eine Wiedergabe der abgespeicherten Pose), das Herstellen, Nutzen, und/oder Trennen einer Kommunikationsverbindung zwischen der chirurgischen Manipulatorvorrichtung und einem externen Eingabesystem, insbesondere OP-Navigationssystem, ob eine translatorische Bewegung oder rotatorische Bewegung (Schwenk- und/oder Neigebewegung) eines aufgenommenen Instruments ausgeführt wird, Abstand zwischen einem aufgenommenen Instrument und einer Zielposition, eine aus einem internen Speicher ausgewählte und anzufahrende vorabgespeicherte Pose der ersten und zweiten Lenkeranordnungen (z.B. eine Wiedergabe der ausgewählten Pose), einen Ort eines Pivotpunkts eines aufgenommenen chirurgischen Instruments, einen Skalierungsgrad für eine Bewegung eines aufgenommenen chirurgischen Instruments.

Bevorzugt ist die Anzeigeeinrichtung dazu ausgebildet, einen Arbeitsraum des Manipulators darzustellen. Dabei zeigt die Anzeigeeinrichtung auf eine Oberseite des Gehäuses (bezogen auf eine Initialstellung des Manipulators) den Arbeitsraum für Schwenk- und Neigebewegung sowie für ein paralleles Bewegen des Instruments in der Ebene an. Auch können eine obere und untere Arbeitsraumgrenze der Instrumentenaufnahmeeinrichtung über die Anzeigeeinrichtung unter Verwendung eines entsprechendes Musters, einer Blink-Frequenz, einer Farbe, und/oder einer Intensität der Beleuchtung dargestellt werden. Für die obere Arbeitsraumgrenze der Instrumentenaufnahmeeinrichtung wird vorzugsweise ein oberes Anzeigesegment, für die untere Arbeitsraumgrenze ein unteres Anzeigesegment, das an einer unteren Seite des Gehäuses (bezogen auf die Initialstellung) abgeordnet ist, entsprechend beleuchtet. Der Anwender kann dann sehr leicht erkennen, ob und wie viel verfügbarer Arbeitsraum zur Verfügung steht.

Vorzugsweise ist die Anzeigeeinrichtung dazu eingerichtet, anzuzeigen, wie der Arbeitsraum der chirurgischen Manipulatorvorrichtung relativ zu seinem eigenen Ursprungkoordinatensystem ausgerichtet ist. Beispielsweise muss der Arbeitsraum bei gleicher Ausrichtung der chirurgischen Manipulatorvorrichtung relativ zu einem Patienten rotiert werden, sodass eine Ausrichtung eines aufgenommenen chirurgischen Instruments zum Patienten sinnvoll und anatomisch korrekt positioniert ist. Hierfür kann unter Umständen ein gewinkelter Instrumentenadapter zum Einsatz kommen der eine entsprechende Winkelkorrektur aufweist. Indem die Koordinatentransformation angezeigt wird, ist ein Bediener über die Ausrichtung stets informiert.

Vorzugsweise weist die Anzeigeeinrichtung wenigstens zwei, vorzugsweise wenigstens vier Anzeigesegmente auf, wobei jedes Anzeigesegment einem Paar oder einem einzigen der vier Hebelgelenkpunkte der ersten und/oder zweiten Lenkeranordnung zugeordnet ist. Beispielsweise ist ein erstes Anzeigesegment den ersten und zweiten Hebelgelenkpunkten der ersten und zweiten Hebel zugeordnet und ein zweites Anzeigesegment den dritten und vierten Hebelgelenkpunkten der dritten und vierten Hebel zugeordnet. Dadurch kann die Anzeigeeinrichtung beispielsweise anzeigen, dass sich die ersten und zweiten Hebel bewegen, während die dritten und vierten Hebel stillstehen. Weist die Anzeigeeinrichtung vier Anzeigesegmente auf, kann die Anzeigeeinrichtung auch dazu verwendet werden, die Bewegung der fünften bis achten Hebel anzuzeigen. Das dritte Segment kann dann dazu verwendet werden, eine Bewegung der fünften und sechsten Hebel anzuzeigen, während das vierte Anzeigesegment eine Bewegung der siebten und achten Hebel anzeigt. Hierdurch ist ein Bediener stets darüber informiert, ob sich die ersten und zweiten Lenkeranordnungen bewegen und wenn ja, in welche Richtung bzw. welches Segment der Lenkeranordnungen sich bewegt. Der Bediener kann so erkennen, ob sich das Instrument bewegt und in welche Richtung sich das Instrument bewegt.

Vorzugsweise weist die Anzeigeeinrichtung eine oder mehrere ringförmige Anzeigen auf. Vorzugsweise umschließt sie ringförmig die vier Hebelgelenkpunkte. Hierdurch ist eine einfache optische Zuordnung der Anzeigeeinrichtung und insbesondere einzelner Anzeigesegmente der Anzeigeeinrichtung zu Hebelgelenkpunkten möglich und eine intuitive Erfassung durch den Bediener erreicht. Es ist auch denkbar, dass je Hebelgelenkpunk eine separate ringförmige Anzeige koaxial zur Gelenkachse vorgesehen ist.

Weiterhin ist bevorzugt, dass die Anzeigeeinrichtung dazu eingerichtet ist, eine Bewegung wenigstens eines Teils der ersten und zweiten Lenkeranordnungen um einen entsprechenden Hebelgelenkpunkt anzuzeigen.

Ferner ist ein Verfahren zum Positionieren eines chirurgischen Instruments, insbesondere Endoskops, mittels einer chirurgischen Manipulatorvorrichtung, insbesondere nach einer der vorstehend beschriebenen bevorzugten Ausführungsformen einer chirurgischen Manipulatorvorrichtung gemäß dem ersten Aspekt der Erfindung offenbart, mit den Schritten: Ermitteln eines ersten Vektors für einen ersten Halter zum Halten einer Instrumentenaufnahme für das chirurgische Instrument, der innerhalb einer ersten Bewegungsebene liegt; Ermitteln eines zweiten Vektors für einen zweiten Halter zum Halten der Instrumentenaufnahme für das chirurgische Instrument, der innerhalb einer zweiten Bewegungsebene liegt; Bewegen des ersten Halters in Übereinstimmung mit dem ersten Vektor mittels einer ersten an dem Rahmen gelagerten Lenkeranordnung, welche den Rahmen mit dem ersten Halter gelenkig verbindet; und Bewegen des zweiten Halters in Übereinstimmung mit dem zweiten Vektor mittels einer zweiten Lenkeranordnung, welche den Rahmen mit dem zweiten Halter gelenkig verbindet, wobei die ersten und zweiten Bewegungsebenen stets parallel zueinander sind. Es soll verstanden werden, dass die Schritte des Verfahrens gleichzeitig oder nacheinander ausgeführt werden können und deren Reihenfolge nicht zwangsläufig der beschriebenen entsprechen muss. Es soll weiterhin verstanden werden, dass die chirurgische Manipulatorvorrichtung gemäß dem ersten Aspekt der Erfindung und das Verfahren gleiche und ähnliche bevorzugte Weiterentwicklungen umfassen. Insofern wird vollumfänglich auf die obige Beschreibung der chirurgischen Manipulatorvorrichtung des ersten Aspekts der Erfindung Bezug genommen.

Vorzugsweise umfasst das Verfahren auch die Schritte Ermitteln einer Geschwindigkeit und Sequenz, mit der der erste und zweite Halter in Übereinstimmung mit dem ersten und zweiten Vektor bewegt werden. Weiterhin umfasst das Verfahren vorzugsweise die Schritte: Ermitteln einer ersten Drehung für den ersten Halter; Ermitteln einer zweiten Drehung für den zweiten Halter; Drehen des ersten Halters in Übereinstimmung mit der ersten Drehung mittels der ersten an dem Rahmen gelagerten Lenkeranordnung; und Drehen des zweiten Halters in Übereinstimmung mit der zweiten Drehung mittels der zweiten Lenkeranordnung; wobei Drehachsen der Drehungen senkrecht zu den ersten und zweiten Bewegungsebenen angeordnet sind. Es soll verstanden werden, dass die Drehachsen des ersten und zweiten Halters senkrecht zu den Bewegungsebenen sind. Die Drehachse einer an den Haltern aufgenommenen Instrumentenaufnahme muss hingegen nicht zwangsläufig senkrecht zu den Bewegungsebenen angeordnet sein; sie kann vielmehr auch schräg verlaufen. Vorzugsweise erfolgt das Bewegen in Übereinstimmung mit den ersten und zweiten Vektoren sowie das Drehen in Übereinstimmung mit den ersten und zweiten Drehungen automatisiert, insbesondere mittels eines elektrischen Antriebs, der in der chirurgischen Manipulatorvorrichtung vorgesehen ist.

Weiterhin ist bevorzugt, dass das Verfahren die Schritte aufweist: Aufnehmen eines ersten chirurgischen Instruments an der Instrumentenaufnahme; und Erfassen eines Pivotpunkts des ersten chirurgischen Instruments bezogen auf ein Objekt durch Anfahren des Pivotpunkts mittels der ersten und zweiten Halter und Abspeichern dieser Position in einem Speicher. Das erste chirurgischen Instrument kann in diesem Fall ein auch eine Pivotpunktlehre, die zum Ermitteln des Pivotpunkts vorgesehen ist, oder ein Tasterinstrument. Als Pivotpunkt wird allgemein ein Punkt des chirurgischen Instruments verstanden, der bezogen auf ein Objekt, wie zum Beispiel dem Patient, im Wesentlichen fix ist oder sich abhängig vom Objekt ändert und um den das Instrument während einer Untersuchung oder Operation gedreht werden muss. So ist beispielsweise das Endoskop bei der HNO-Chirurgie um einen bestimmten Pivotpunkt, der insbesondere etwa im Eingangsbereich des Nasenvorhofs liegt, zu drehen, im Wesentlichen unabhängig von der Eindringtiefe des Endoskops in die Nasenhaupthöhle hinein.

Dieser Pivotpunkt wird vorzugsweise in einem Speicher abgespeichert. Vorzugsweise werden auch Vorschriften abgespeichert, die eine Änderung des Pivotpunkts, zum Beispiel abhängig von einer Eindringtiefe des chirurgischen Instruments in den Körper des Patienten, beschreiben. Der Speicher kann in der chirurgischen Manipulatorvorrichtung vorgesehen sein oder entfernt von dieser. Durch Speichern des Pivotpunkts kann dieser in nachfolgenden Operationen oder Ermittlung von Bewegungsbahnen, Trajektorien und dergleichen verwendet werden. Indem der Pivotpunkt mittels des Instuments bzw. mittels Positionierung der ersten und zweiten Halter angefahren wird, ist die Stellung der ersten und zweiten Lenkeranordnung mit einem Pivotpunkt bekannt. Das Anfahren eines Pivotpunkts kann einerseits manuell erfolgen, indem ein Operateur das Instrument zu dieser Stelle hinführt und die ersten und zweiten Lenkeranordnungen in diesem Fall passiv arbeiten. Eine weitere Möglichkeit ist auch ein manuell gesteuertes Anfahren, bei dem ein Operateur das Instrument beispielsweise mittels einer Art Joystick oder einer anderen Bedieneinheit führt, um das Instrument an den Pivotpunkt zu führen.

Gemäß einer weiteren bevorzugten Ausführungsform des Verfahrens wird das Ermitteln der ersten und zweiten Vektoren unter Verwendung des gespeicherten Pivotpunkts ausgeführt. Vorzugsweise ist der chirurgische Manipulator in einen Pivotpunkt-Modus und in einen Normalmodus versetzbar. In dem Pivotpunkt-Modus werden sämtliche Bewegungsbahnen und Trajektorien, insbesondere die ersten und zweiten Vektoren sowie die sequentielle Abfolge der Bewegung der ersten und zweiten Lenkeranordnungen in Übereinstimmung mit den ersten und zweiten Vektoren unter Berücksichtigung des Pivotpunkts ausgeführt. Das heißt, auch bei einer Verschwenkung oder anderen Bewegung des Instruments wird dieses stets um den Pivotpunkt bewegt. Im Normalmodus hingegen, wird der Pivotpunkt nicht berücksichtigt. Das Instrument kann beispielsweise auch außerhalb des Pivotpunkts bewegt werden. In diesem Modus ist es beispielsweise denkbar, die ersten und zweiten Lenkeranordnung mit Relativbewegungen zueinander zu bewegen, die in einem Verhältnis von 1:1 stehen. Das heißt, die Instrumentenaufnahme wird in diesem Fall nicht verschwenkt, sondern nur linear bewegt. Das Versetzen des chirurgischen Manipulators in den Pivotpunkt-Modus und den Normalmodus erfolgt vorzugsweise in Antwort auf ein Signal, welches beispielsweise mittels eines Schalters an der chirurgischen Manipulatorvorrichtung oder einem entfernt angeordneten Schalter, beispielsweise einem Fußpedal, durch einen Bediener ausgelöst wird. Auch ist es denkbar, dass dieses Signal von einer übergeordneten Steuereinheit, wie beispielsweise einem OP-Navigationssystem, an die chirurgische Manipulatorvorrichtung übertragen wird. Dies kann drahtgebunden oder drahtlos erfolgen.

In einer weiteren bevorzugten Ausführungsform des Verfahrens umfasst dieses die Schritte: Empfangen eines Signals, das eine Anfahranforderung des Pivotpunkts repräsentiert; und Ermitteln des ersten und zweiten Vektors derart, dass ein aufgenommenes Instrument an dem Pivotpunkt positioniert wird. Bevorzugt ist, dass das Abspeichern des Pivotpunkts mittels einer Pivotpunkt-Lehre erfolgt. Diese Pivotpunkt-Lehre wird nach Abspeichern des Pivotpunkts wieder von der Aufnahme entnommen, und ein anderes chirurgisches Instrument, in diesem Fall dann ein Endoskop oder dergleichen, wird an der Instrumentenaufnahme aufgenommen. Um das Endoskop dann mit der Endoskopspitze wieder zum Pivotpunkt zu bringen, dient das Signal, das eine Anfahranforderung des Pivotpunkts repräsentiert. Ein solches Anfahranforderungssignal kann beispielsweise durch einen Schalter oder durch ein anderes externes Signal ausgelöst werden. Die Ermittlung der ersten und zweiten Vektoren erfolgt dann derart, dass das aufgenommene Instrument mit der Instrumentenspitze an dem Pivotpunkt positioniert wird. Hierzu kann auch vorgesehen sein, dass eine axiale Erstreckung wenigstens teilweise senkrecht zu den ersten und zweiten Bewegungsebenen berücksichtigt wird. Das Anfahren des Pivotpunkts umfasst in diesem Fall vorzugsweise auch ein Betätigen eines Linearantriebs einer Instrumentenaufnahmeeinrichtung.

Gemäß einem weiteren Aspekt des Verfahrens umfasst dieses den Schritt: Ermitteln einer Transformationsmatrix zwischen einer Basis der chirurgischen Manipulatorvorrichtung und dem Pivotpunkt. Neben den Koordinaten des Pivotpunkts wird vorzugsweise eine Transformationsmatrix ermittelt, die dann neben den Punktkoordinaten auch die aktuelle Orientierung des chirurgischen Instruments enthält, wodurch ein Koordinatensystem im Pivotpunkt dargestellt werden kann. Dies ist insbesondere vorteilhaft für Visualisierungen oder weitere Raumtransformationen relativ zum Pivotpunkt. Die PivotpunktKoordinaten bzw. die Transformationsmatrizen werden vorzugsweise relativ zu einem Basiskoordinatensystem definiert, welches von der chirurgischen Manipulatorvorrichtung oder besonders bevorzugt von einer Haltevorrichtung, beispielsweise einem Haltearm oder einem Stativ, an dem die chirurgische Manipulatorvorrichtung angebracht ist, definiert wird. Ein solches Basiskoordinatensystem ist vorzugsweise auf einen OP-Tisch bezogen.

Vorzugsweise werden die Koordinaten des Pivotpunkts und/oder die Transformationsmatrix an einer Schnittstelle bereitgestellt, beispielsweise über eine RESTful-API-Schnittstelle.

In einer weiteren Ausführungsform des Verfahrens ist das chirurgische Instrument vorzugsweise als ein Tastinstrument ausgebildet, und das Verfahren umfasst die Schritte: Anfahren einer ersten anatomischen Landmarke eines Patienten mittels des Tastinstruments; Abspeichern von ersten Landmarken-Daten, die die Pose der ersten und zweiten Lenkeranordnungen und/oder die Position der ersten und zweiten Halter repräsentieren; Verknüpfen der ersten Landmarken-Daten mit ersten Bilddaten, die wenigstens eine voraufgenommene Schichtaufnahme des Patienten repräsentieren. Solche voraufgenommenen oder präoperativ erfassten Schichtaufnahmen werden bei Operationen üblicherweise von dem Patienten erstellt. Solche Schichtaufnahmen können beispielsweise von einem CT-Gerät erfasst werden. Das Verfahren umfasst ferner vorzugsweise die Schritte Anfahren einer zweiten anatomischen Landmarke eines Patienten mittels des Tastinstruments; Abspeichern von zweiten Landmarken-Daten, die die Pose der ersten und zweiten Lenkeranordnungen und/oder die Position der ersten und zweiten Halter an der zweiten anatomischen Landmarke des Patienten repräsentieren; und Verknüpfen der zweiten Landmarken-Daten mit zweiten Bilddaten, die wenigstens eine voraufgenommene Schichtaufnahme des Patienten repräsentieren. Ebenso kann mit dritten, vierten und fünften anatomischen Landmarken oder weiteren verfahren werden. Auf diese Weise können spätere intra-operative Kollisionen zwischen dem chirurgischen Instrument und umliegendem Gewebe vermieden werden und eine geplante Bewegungsbahn bzw. Trajektorie entsprechend umgesetzt werden.

Ferner ist ein Ankopplungselement für eine chirurgische Manipulatorvorrichtung offenbart, insbesondere eine chirurgische Manipulatorvorrichtung nach einer der vorstehend beschriebenen bevorzugten Ausführungsformen einer chirurgischen Manipulatorvorrichtung gemäß dem ersten Aspekt der Erfindung, wobei das Ankopplungselement das Ankoppeln eines chirurgischen Instruments an eine Instrumentenaufnahmeeinrichtung erlaubt, wobei das Ankopplungselement einen Hauptkörper aus einem flexiblen elektrisch isolierenden Material aufweist, wobei der Hauptkörper Formschlussmittel zur Kopplung mit der Instrumentenaufnahmeeinrichtung und einen Klemmabschnitt zum klemmenden Koppeln mit dem chirurgischen Instrument aufweist.

Ferner ist eine Instrumentenaufnahmeeinrichtung für eine chirurgische Manipulatorvorrichtung, insbesondere nach einer der vorstehend beschriebenen bevorzugten Ausführungsformen einer chirurgischen Manipulatorvorrichtung gemäß dem ersten Aspekt der Erfindung und zum Aufnehmen eines chirurgischen Instruments offenbart, wobei die Instrumentenaufnahmeeinrichtung einen Linearantrieb aufweist, zum Positionieren des Instruments, wobei der Linearantrieb eine längliche Hülse, einen in der Hülse angeordneten Spindeltrieb und ein Abtriebselement aufweist, das die Formschlussmittel trägt, wobei der Spindeltrieb einen in der Hülse angeordneten Magnetmitnehmer antreibt und das Abtriebselement äußerlich und linear verschieblich an der Hülse gelagert ist und mittels magnetischer Kraft mit dem Magnetmitnehmer gekoppelt ist.

Nachstehend wird die Erfindung anhand eines Ausführungsbeispiels unter Bezugnahme auf die beliegenden Figuren näher erläutert. Dabei zeigen:
- Fig. 1: eine perspektivische Ansicht eines robotischen Haltearms mit einer chirurgischen Manipulatorvorrichtung gemäß der Erfindung;
- Fig. 2: eine perspektivische Ansicht der chirurgischen Manipulatorvorrichtung;
- Fig. 3: eine Seitenansicht der chirurgischen Manipulatorvorrichtung gemäß Fig. 2;
- Fig. 4: eine Draufsicht auf die chirurgische Manipulatorvorrichtung gemäß Fig. 3, aber ohne chirurgisches Instrument;
- Fig. 5: eine Unteransicht der chirurgischen Manipulatorvorrichtung gemäß Fig. 4;
- Fig. 6: die chirurgische Manipulatorvorrichtung gemäß Fig. 4 in einer zweiten Pose;
- Fig. 7: die chirurgische Manipulatorvorrichtung gemäß Fig. 4 in einer dritten Pose;
- Fig. 8: die chirurgische Manipulatorvorrichtung gemäß Fig. 4 in einer vierten Pose;
- Fig. 9: die chirurgische Manipulatorvorrichtung gemäß Fig. 4 in einer fünften Pose;
- Fig. 10: eine vergrößerte Darstellung der Kinematik der chirurgischen Manipulatorvorrichtung;
- Fig. 11: eine vergrößerte Darstellung eines Hebels;
- Fig. 12: eine vergrößerte Darstellung eines Lenkers;
- Fig. 13: eine vergrößerte Darstellung eines Stabs;
- Fig. 14: eine vergrößerte Darstellung eines Halters;
- Fig. 15: eine vergrößerte Darstellung eines Kardanelements;
- Fig. 16: eine Seitenansicht der chirurgischen Manipulatorvorrichtung ohne Gehäuse und mit Antrieb;
- Fig. 17: eine Seitenansicht der chirurgischen Manipulatorvorrichtung ohne Gehäuse mit Bremsen für die Kinematik;
- Fig. 18: eine Seitenansicht einer Instrumentenaufnahmeeinrichtung;
- Fig. 19: einen Vollschnitt durch die Instrumentenaufnahmeeinrichtung aus Fig. 18;
- Fig. 20: eine erste Ansicht eines Ankopplungselements samt Adapter;
- Fig. 21: eine zweite Ansicht des Ankopplungselements aus Fig. 20;
- Fig. 22: eine schematische Darstellung der Manipulatorvorrichtung mit Peripheriegeräten;
- Fig. 23: eine perspektivische Ansicht der chirurgischen Manipulatorvorrichtung mit einer Pivotpunktlehre aufgenommen;
- Fig. 24: eine schematische Darstellung der Pivotpunkt-Steuerung;
- Fig. 25: eine schematische Darstellung einer Rotation des chirurgischen Instruments um den Pivotpunkt; und

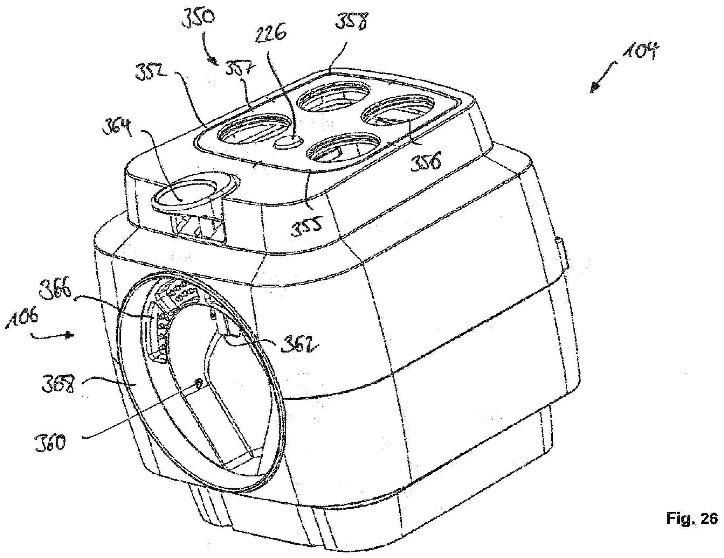

- Fig. 26: eine perspektivische Ansicht eines Gehäuses der chirurgischen Manipulatorvorrichtung mit Anzeigeeinrichtung;

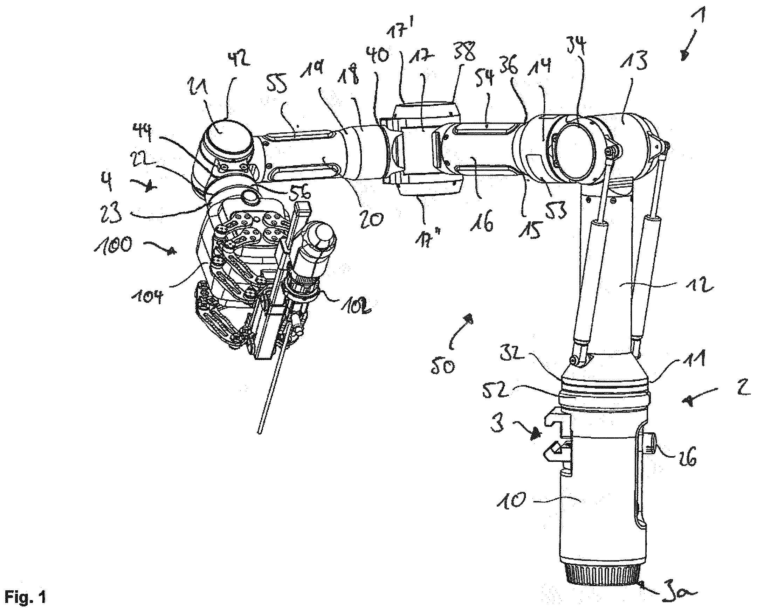

Gemäß Figur 1 ist eine Haltevorrichtung 1, in Form eines robotischen Haltearms gezeigt, an dem eine chirurgische Manipulatorvorrichtung 100 aufgenommen ist. Die Haltevorrichtung weist ein proximales Ende 2 zur Befestigung der Haltevorrichtung 1 an einer Basis (nicht gezeigt) auf. Die Basis kann gemäß diesem Ausführungsbeispiel als Normschiene eines Operationstisches ausgebildet (der Operationstisch ist in Figur 1 nicht gezeigt). Daher weist die Haltevorrichtung 1 am proximalen Ende 2 eine Klemmklaue 3 auf, die sich über eine Schraube 3a manuell zuspannen lässt. Die Haltevorrichtung 1 weist ferner ein distales Ende 4 zum Aufnehmen eines Anbaugeräts auf, welches hier als chirurgische Manipulatorvorrichtung 100 gemäß der Erfindung ausgebildet ist.

Die Haltevorrichtung gemäß Figur 1 weist sieben Armsegmente 10, 12, 14, 16, 18, 20, 22 auf, wobei zwischen den einzelnen Armsegmenten 10 bis 22 die Gelenke 11, 13, 15, 17, 19, 21, 23 vorgesehen sind. Das erste Armsegment 10 bildet das proximale Ende 2 und weist die Klemmklaue 3 auf. An dem Armsegment 10 ist ferner ein Einschaltknopf 26 vorgesehen zum Einschalten der gesamten Haltevorrichtung, zwei Anschlüsse, über die die Haltevorrichtung mit Strom und Daten, wie beispielsweise Stellsignale und dergleichen, versorgbar ist, und über die die Daten von der Haltevorrichtung an externe Einheiten, wie an OP-Systeme übertragen werden, sowie ein Notausschalter.

Die Gelenke 11, 15, 19 und 23 sind als rotatorische Gelenke ausgebildet und die Gelenke 13, 17 und 21 als Schwenkgelenke.

Die Haltevorrichtung 1 weist an jedem Gelenk 11, 13, 15, 17, 19, 21, 23 eine Anzeigeeinheit 32, 34, 36, 38, 40, 42, 44 auf, die dazu vorgesehen sind, einen Status der Haltevorrichtung 1 anzuzeigen.

Die Anzeigeeinheiten 32, 34, 36, 38, 40, 42, 44 sind gemäß diesem Ausführungsbeispiel als im Wesentlichen ringförmige Lichtquellen, insbesondere als LED-Ringe ausgebildet. Die Zentralachse von jedem Ring verläuft im Wesentlichen koaxial zur jeweiligen Drehachse des Gelenks 11, 13, 15, 17, 19, 21, 23. Während für die Gelenke 11, 15, 19, 23 jeweils ein einziger LED-Ring vorgesehen ist, sind für die Gelenke 13, 17 und 21 jeweils zwei LED-Ringe vorgesehen. Die zwei LED-Ringe sind an vorderen und hinteren Gelenkabschnitten 17', 17" (in Figur 1 nur beispielsweise mit Bezugszeichen versehen) vorgesehen. So ist in jeder Lage der Haltevorrichtung jede Anzeigeeinheit stets erkennbar.

Gemäß diesem Ausführungsbeispiel weist die Haltevorrichtung ferner eine Bedieneinrichtung 50 auf. Mittels der Bedieneinrichtung 50 ist der Haltearm in eine gewünschte Pose verbringbar, wobei die Bedieneinrichtung 50 dazu eingerichtet ist, bei Kontakt zwischen einer Bedienperson und einem der sieben Armsegmente 10, 12, 14, 16, 18, 20, 22, das zugeordnete Gelenk 11, 13, 15, 17, 19, 21, 23 freizugeben. Dazu weist die Bedieneinrichtung 50 gemäß diesem Ausführungsbeispiel fünf Kontaktabschnitte 52, 53, 54, 55, 56 auf, wobei jeder Kontaktabschnitt 52, 53, 54, 55, 56 an einem anderen Armsegment 11, 14, 16, 20, 22 angeordnet ist. Die einzelnen Kontaktabschnitte sind als berührungsempfindliche Oberflächen bzw. Taster ausgebildet, sodass bei Kontakt zwischen einer Bedienperson und einem entsprechenden Kontaktabschnitt ein oder mehrere zugeordnete Gelenke freigegeben werden.

Die Zuordnung der einzelnen Gelenke 11, 13, 15, 17, 19, 21, 23 ist gemäß diesem Ausführungsbeispiel wie folgt geregelt: Bei Kontakt zwischen einer Bedienperson und dem Armsegment 10, also dem Kontaktabschnitt 52 wird das Gelenk 11 freigegeben. Eine Bedienperson kann nun einen Freiheitsgrad beeinflussen. Kommt eine Bedienperson mit dem Armsegment 14 in Kontakt, wird das Gelenk 15 freigegeben, bei Kontakt mit dem Kontaktmittel 54 das Gelenk 13, bei Kontakt mit dem Kontaktmittel 55 die Gelenke 19 und 17, und bei Kontakt mit dem Kontaktmittel 56 die Gelenke 23 und 21. Hierbei ist bevorzugt vorgesehen, dass die entsprechenden Anzeigeeinheiten 32, 34, 36, 40, 42, 44 dieses Freigeben anzeigen, also insbesondere durch Aufleuchten des LED-Rings.

Der genaue Aufbau der Haltevorrichtung und seine Funktion ist in DE 10 2014 016 823 A1, DE 10 2014 016 824 A1, DE 10 2015 104 810 A1, DE 10 2015 104 819 A1 und EP 3 130 305 A1 im Detail beschrieben. Der Offenbarungsgehalt dieser Druckschriften bezüglich der Haltevorrichtung 1 wird vollumfänglich hier durch Referenz mit einbezogen.

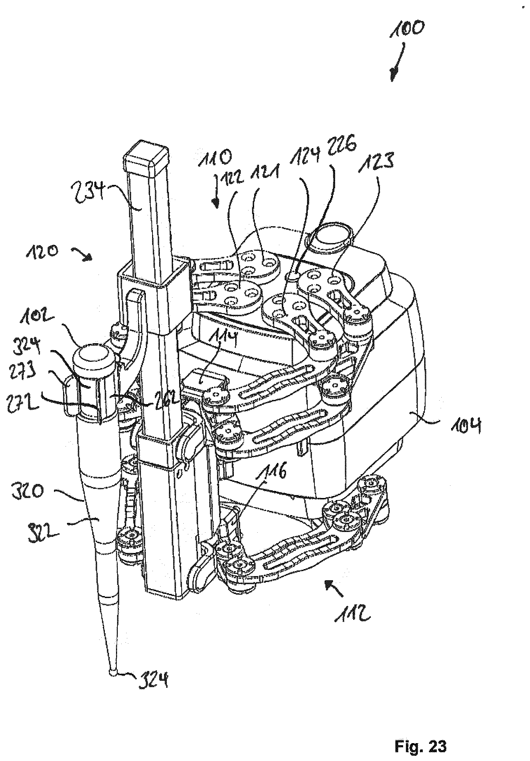

Die chirurgische Manipulatorvorrichtung 100 hält in diesem Ausführungsbeispiel als chirurgisches Instrument 102 ein Endoskop. Die chirurgische Manipulatorvorrichtung 100 weist ein Gehäuse 104 auf, das eine Schnittstelle 106 hat (vgl. Fig. 23), über die die chirurgische Manipulatorvorrichtung 100 mit dem distalen Ende 4 der Haltevorrichtung 1 gekoppelt ist. Die Schnittstelle 106 wird im Folgenden mit Bezug auf Fig. 23 noch näher erläutert werden.

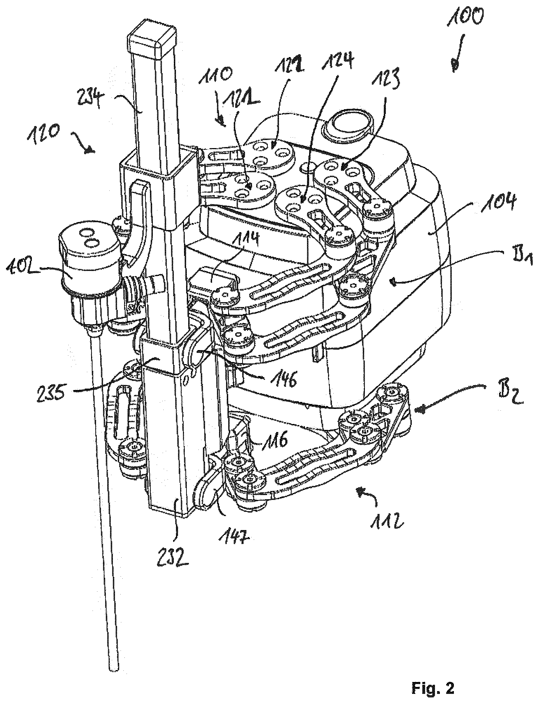

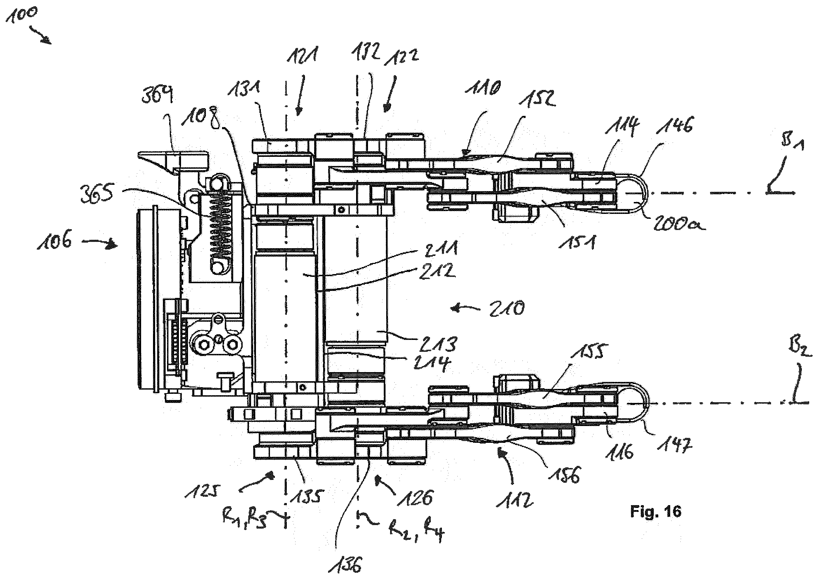

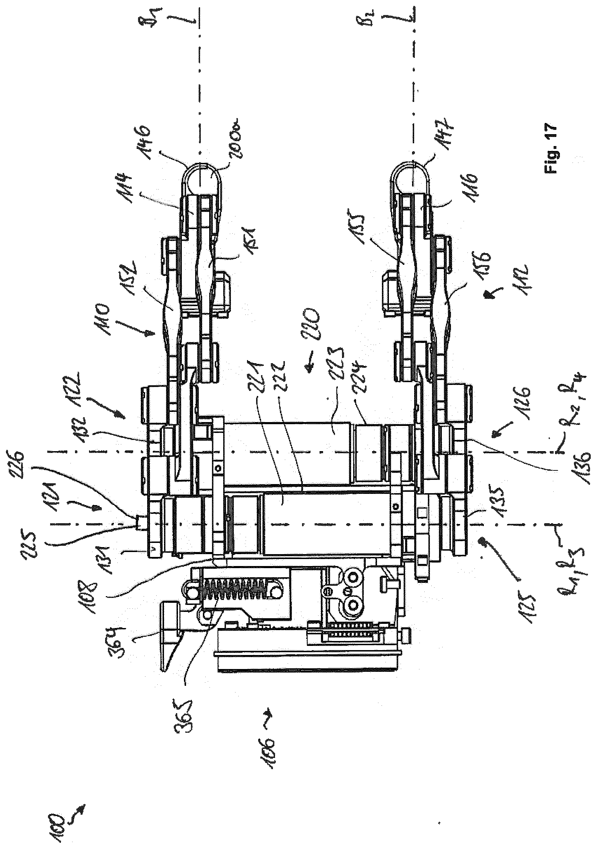

Die chirurgische Manipulatorvorrichtung 100 (im Folgenden auch nur als "Manipulatorvorrichtung 100" bezeichnet) weist darüber hinaus einen Rahmen 108 auf (vgl. Fig. 16 und 17), der eine Struktur der Manipulatorvorrichtung 100 definiert. Der Rahmen ist in Fig. 2 nicht zu sehen, da dieser von dem Gehäuse 104 umgeben ist.

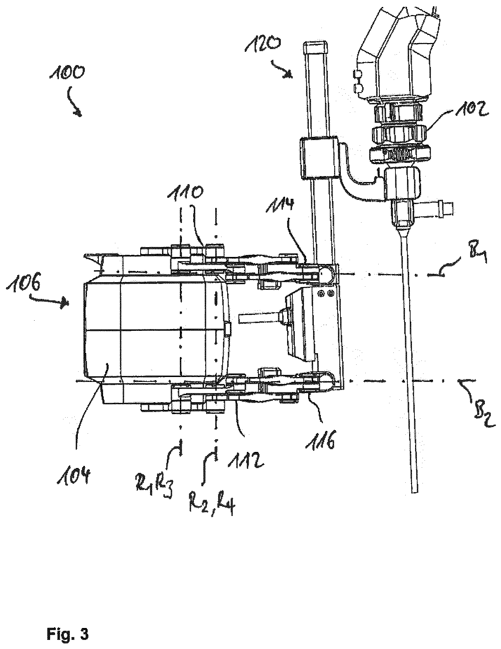

An dem Rahmen 108 sind eine erste Lenkeranordnung 110 und eine zweite Lenkeranordnung 112 gelagert. Die erste Lenkeranordnung 110 ist in einer erste Bewegungsebene B1 bewegbar, und die zweite Lenkeranordnung 112 in einer zweiten Bewegungsebene B2 (vgl. auch Fig. 3). Die Bewegungsebenen B1, B2 sind parallel zueinander und nicht gegeneinander verkippbar.

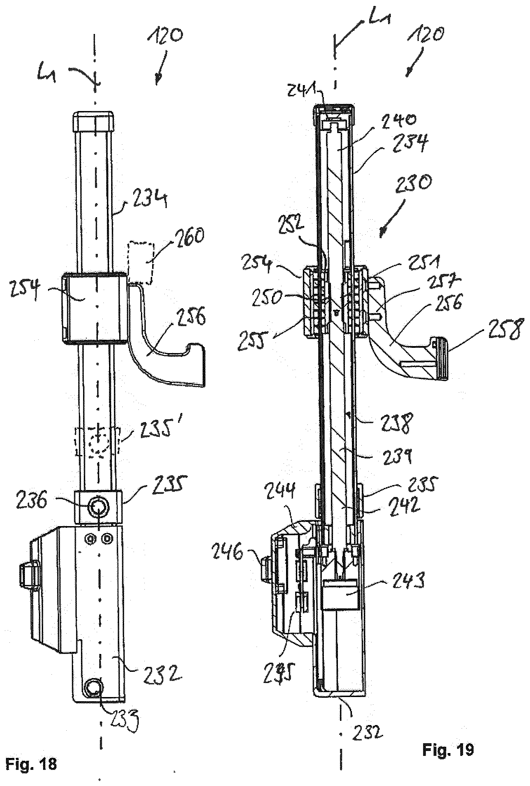

Die erste Lenkeranordnung 110 verbindet den Rahmen 108 mit einem ersten Halter 114, und die zweite Lenkeranordnung 112 verbindet den Rahmen mit einem zweiten Halter 116. Mittels der Halter 114, 116 ist in diesem Ausführungsbeispiel (Fig. 2 und 3) eine Instrumentenaufnahmeeinrichtung 120 befestigt, wie dies im Detail mit Bezug auf Fig. 18 und 19 näher erläutert werden wird.

Es kann auch Ausführungsführungen geben, in denen ein Instrument direkt mit den ersten und zweiten Lenkeranordnungen 110, 112 verbunden ist, ohne die Zwischenschaltung einer Instrumentenaufnahmeeinrichtung 120. Insbesondere ist es auch denkbar, dass ein chirurgisches Instrument 102 integral mit den ersten und zweiten Haltern 114, 116 gebildet ist, insbesondere stoffschlüssig und nicht zerstörungsfrei von diesen lösbar ist. Für einen solchen Fall kann es bevorzugt sein, zwischen den ersten und zweiten Haltern 114, 116 und den entsprechenden ersten und zweiten Lenkeranordnungen 110, 112 eine Clipverbindung oder dergleichen vorzusehen, um so das chirurgische Instrument 102 der chirurgischen Manipulatorvorrichtung 100 auswechseln zu können.

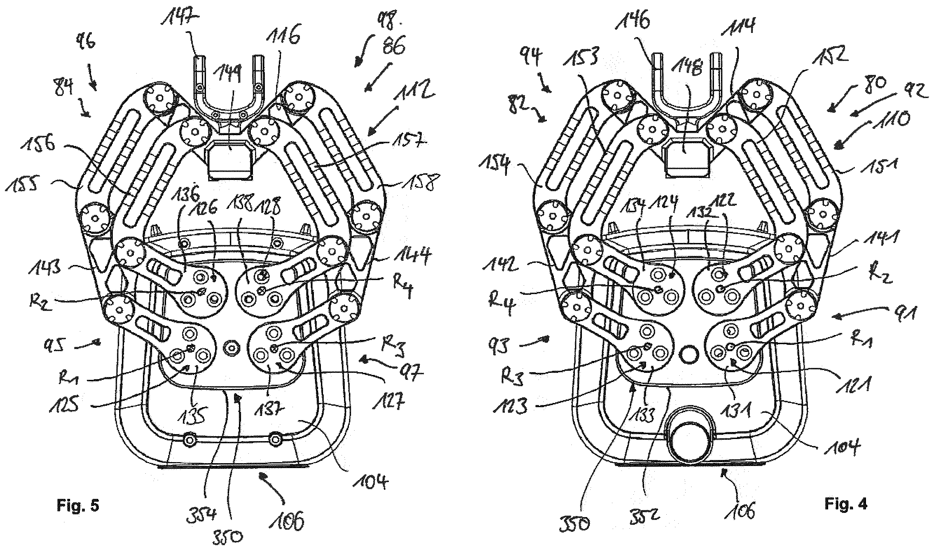

Die erste Lenkeranordnung 110 ist an vier Hebelgelenkpunkten 121, 122, 123, 124 der ersten Lenkeranordnung 110 mit dem Rahmen 108 gekoppelt, und die zweite Lenkeranordnung 112 ist an vier Hebelgelenkpunkten 125, 126, 127, 128 der zweiten Lenkeranordnung mit dem Rahmen 108 gekoppelt (vgl. auch Fig. 4, 5).

Der erste Hebelgelenkpunkt 121 weist eine erste Rotationsachse R1, der zweite Hebelgelenkpunkt 122 eine zweite Rotationsachse R2, der dritte Hebelgelenkpunkt 123 eine dritte Rotationsachse R3 und der vierte Hebelgelenkpunkt 124 eine vierte Rotationsachse R4 auf. Die vier Hebelgelenkpunkte 125, 126, 127, 128 der zweiten Lenkeranordnung 112 sind als fünfter Hebelgelenkpunkt 125, sechster Hebelgelenkpunkt 126, siebter Hebelgelenkpunkt 127 und achter Hebelgelenkpunkt 128 bezeichnet. In diesem Ausführungsbeispiel (vgl. Fig. 3, 4 und 5) weisen die vier Hebelgelenkpunkte 121, 122, 123, 124 der ersten Lenkeranordnung 110 gemeinsame Rotationsachsen R1, R2, R3, R4 mit den vier zweiten Hebelgelenkpunkten 125, 126, 127, 128 der zweiten Lenkeranordnung 112 auf. Insofern hat der fünften Hebelgelenkpunkt 125 die Rotationsachse R1, der sechste Hebelgelenkpunkt 126 die Rotationsachse R2, der siebte Hebelgelenkpunkt 127 die Rotationsachse R3 und der achte Hebelgelenkpunkt 128 die Rotationsachse R4.

Die ersten und zweiten Lenkeranordnungen 110, 112 sind in diesem Ausführungsbeispiel identisch ausgebildet, aber spiegelsymmetrisch, wie sich insbesondere aus den Fig. 3, 4 und 5 entnehmen lässt; in der Unteransicht (Fig. 5) sieht die zweite Lenkeranordnung 112 identisch aus zu der ersten Lenkeranordnung 110 der Draufsicht gemäß Fig. 4.

Die erste Lenkeranordnung 110 weist erste und zweite Armsegmente 80, 82 auf, die in sich wiederum identisch und spiegelsymmetrisch ausgebildet sind (vgl. Fig. 4). Die zweite Lenkeranordnung 112 weist entsprechende erste und zweite Armsegmente 84, 86 auf, die wiederum in sich identisch aber spiegelsymmetrisch ausgebildet sind.

Jedes der Armsegmente 80, 82, 84, 86 umfasst zwei Parallelogramme, nämlich ein erstes Parallelogramm 91, ein zweites Parallelogramm 92, ein drittes Parallelogramm 93 und ein viertes Parallelogramm 94. Die zweite Hebelgelenkanordnung 112 weist ein fünftes Parallelogramm 95, ein sechstes Parallelogramm 96, ein siebtes Parallelogramm 97 und ein achtes Parallelogramm 98 auf.

Die erste Lenkeranordnung 110 umfasst einen ersten Hebel 131, einen zweiten Hebel 132, einen dritten Hebel 133 und einen vierten Hebel 134, deren Drehachsen jeweils die Rotationsachsen R1, R2, R3, R4 sind. In entsprechender Weise weist die zweite Lenkeranordnung 112 einen fünften Hebel 135, einen sechsten Hebel 136, einen siebten Hebel 137 und einen achten Hebel 138 auf, deren Drehachsen ebenfalls die Rotationsachsen R1, R2, R3, R4 sind. Es soll verstanden werden, dass es aber auch Ausführungsbeispiele gibt, bei denen die Drehachsen der Hebel 135, 136, 137, 138 und somit auch die Rotationsachsen der fünften, sechsten, siebten und achten Hebelgelenkpunkte 125, 126, 127, 128 parallel versetzt zu den Rotationsachsen R1, R2, R3, R4 sind und insofern eigene, separate vier Rotationsachsen aufweisen. Insbesondere ist es denkbar, dass die Hebelgelenkpunkte 125, 126, 127, 128 in Richtung der Schnittstelle 106 versetzt sind, um so einen initialen Anstellwinkel für das chirurgische Instrument 102 (vgl. Fig. 3) bereitzustellen.

Sämtliche Hebel 131 bis 138 sind abtriebsseitig, das heißt an dem den Hebelgelenkpunkten 121 bis 128 entgegengesetzten Ende, mit einem Lenker 141, 142, 143, 144 verbunden. Die ersten und zweiten Hebel 131, 132 sind abtriebsseitig mit einem ersten Lenker 141 verbunden, die dritten und vierten Hebel 133, 134 sind abtriebsseitig mit einem zweiten Lenker 142 verbunden, die fünften und sechsten Hebel 135, 136 sind abtriebsseitig mit einem dritten Lenker 143 verbunden, und die siebten und achten Hebel 137, 138 sind abtriebsseitig mit einem vierten Lenker 144 verbunden.

Der erste Hebel 131, der zweite Hebel 132, der erste Lenker 141 und der Rahmen 108 bilden zusammen das erste Parallelogramm 91. Der dritte Hebel 133, der vierte Hebel 134, der zweite Lenker 142 und der Rahmen 108 bilden zusammen das dritte Parallelogramm 93. Der fünfte Hebel 135, der sechste Hebel 136, der dritte Lenker 143 und der Rahmen 108 bilden zusammen das fünfte Parallelogramm, und der siebte Hebel 137, der achte Hebel 138, der vierte Lenker 144 und der Rahmen 108 bilden zusammen das siebte Parallelogramm.

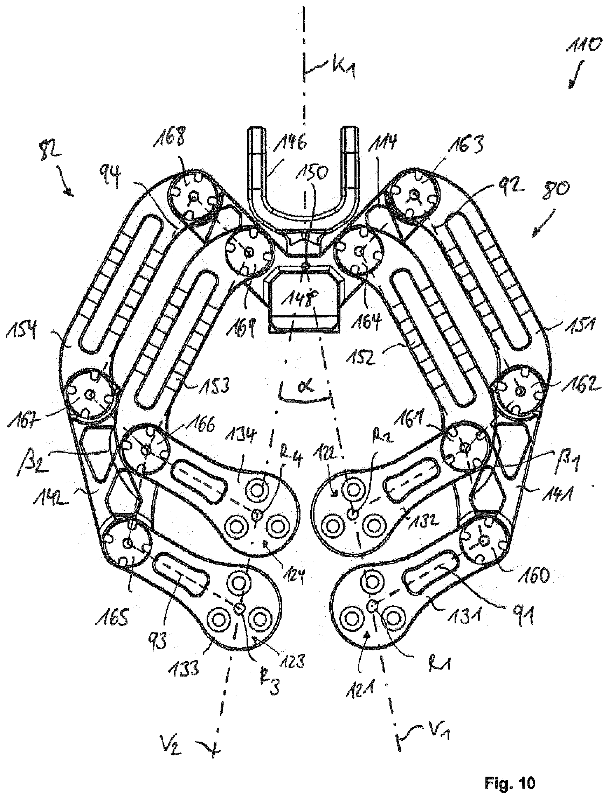

Die erste Lenkeranordnung 110 weist ferner einen ersten Stab 151, einen zweiten Stab 152, einen dritten Stab 153 und einen vierten Stab 154 auf. Die zweite Lenkeranordnung 112 weist einen fünften Stab 155, einen sechsten Stab 156, einen siebten Stab 157 und einen achten Stab 158 auf. Der erste und zweite Stab 151, 152 verbindet den ersten Lenker 141 gelenkig mit dem ersten Halter 114, und der dritte und vierte Stab 153, 154 verbinden den zweiten Lenker 142 gelenkig mit dem ersten Halter 114. In entsprechender Weise verbinden der fünfte Stab 155 und der sechste Stab 156 den dritten Lenker 143 mit dem zweiten Halter 116 und der siebte und achte Stab 157, 158 den vierten Lenker 144 mit dem zweiten Halter 116. Dieser Aufbau wird nochmals mit Bezug auf Fig. 10 im Detail erläutert werden.

An dem ersten Halter 114 ist ferner ein erstes Kardanelement 146 aufgenommen, und an dem zweiten Halter 116 ist ein zweites Kardanelement 147 aufgenommen. Über die Kardanelemente 146, 147 wird die Instrumentenaufnahmeeinrichtung 120 gehalten, wie dies später noch im Detail mit Bezug auf die Figuren 17 und 19 beschrieben wird. Die ersten und zweiten Kardanelemente 146, 147 sind in entsprechenden Gelenkabschnitten 148, 149 der ersten und zweiten Halter 114, 116 drehbar befestigt.

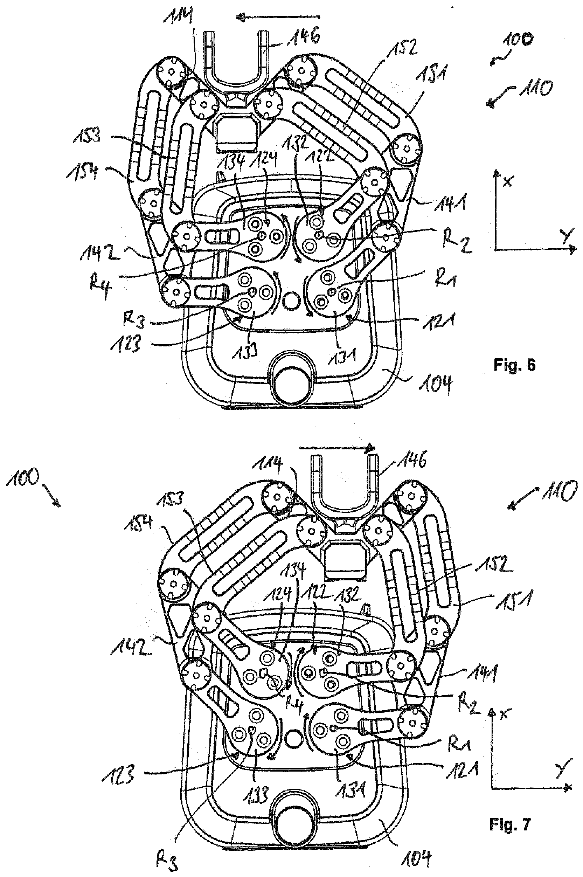

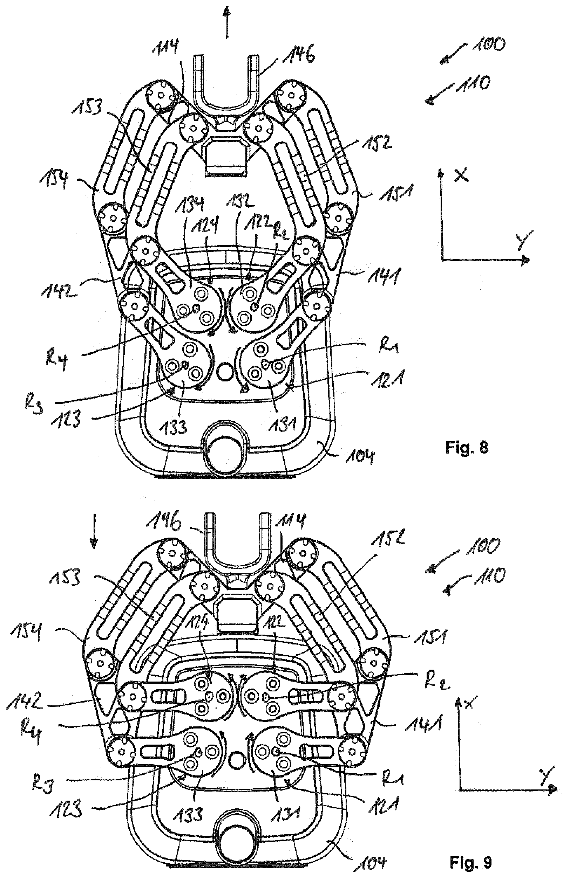

Die Fig. 6 bis 9 illustrieren vier verschiedene Positionen des ersten Halters 114 in der Draufsicht, also in der Ebene B1. Während in Fig. 6 der erste Halter 114 in eine linke Extremposition verschoben ist, ist der erste Halter 114 in Fig. 7 in eine rechte Extremposition, in Fig. 8 in eine vordere Extremposition und in Fig. 9 in eine hintere Extremposition verschoben. Eine Drehung um eine Achse, die senkrecht auf die erste Bewegungsebene B1 ist, ist in den Fig. 6 bis 9 nicht vorgesehen und kann auch aufgrund der hier verwendeten Parallelkinematik nicht umgesetzt werden.

Die Verschiebung zur linken Seite (Fig. 6, vgl. Pfeil oberhalb des Kardanelements 146) wird ausgeführt, indem die ersten und zweiten Hebel 131, 132 linkssinnig bezogen auf die Rotationsachsen R1, R2 rotiert werden, während die dritten und vierten Hebel 133, 134 ebenfalls linkssinnig um die Rotationsachsen R3, R4 rotiert werden. Mit einer entsprechenden Drehung der Hebel 131, 132, 133, 134 in die entgegengesetzte Richtung wird eine Verschiebung des ersten Halters 114 nach rechts mit Bezug auf Fig. 7 bewirkt, wie durch den Pfeil oberhalb des Kardanelements 146 illustriert. Werden die Hebel 131, 132, 133, 134 paarweise gegensinnig bewegt, das heißt die ersten und zweiten Hebel 131, 132 werden mit Bezug auf die dritten und vierten Hebel 133, 134 gegensinnig rotiert, folgt eine Verschiebung des ersten Halters 114 innerhalb der ersten Bewegungsebene B1 in eine x-Richtung mit Bezug auf das gezeigte Koordinatensystem. Dies wird auch durch den Pfeil oberhalb des Kardanelements 146 (Fig. 8) bzw. links neben dem Kardanelement 146 (Fig. 9) angezeigt.

Wie sich aus den Fig. 6 bis 9 leicht erkennen lässt, ist die Aufhängung des ersten Halters 114 in Bezug auf den Rahmen 108 statisch überbestimmt. Zur Positionierung des Halters 114 würden lediglich zwei Hebel, ein Lenker und zwei Stäbe ausreichen. Beispielsweise ist es denkbar, für die Positionierung des Halters 114 nur die Hebel 132, 134 sowie die Stäbe 152, 153 zu verwenden. Ebenso wäre es möglich, nur die Hebel 131, 132, den Lenker 141 und die Stäbe 151, 152 zu verwenden. Durch die Verwendung dieser gezeigten komplexen ersten und zweiten Lenkeranordnungen 110, 112 wird allerdings eine besonders hohe Steifigkeit erreicht und somit eine hohe Positioniergenauigkeit und Wiederholgenauigkeit.

In Fig. 10 ist die erste Lenkeranordnung 110 samt erstem Halter 114 und erstem Kardanelement 146 nochmals vergrößert dargestellt, um die Geometrie besser zu beschreiben. Wiederum sind die ersten, zweiten, dritten und vierten Hebel 131, 132, 133, 134, die ersten und zweiten Lenker 141, 142 und die ersten, zweiten, dritten und vierten Stäbe 151, 152, 153, 154 gezeigt. Weiterhin ist der erste Halter 114 sowie das erste Kardanelement 146 gezeigt. Das erste Kardanelement 146 ist mit der Aufnahme 148 gekoppelt und drehbar um eine Achse K1, die innerhalb der ersten Bewegungsebene B1 liegt. Sie kann aber auch parallel zu dieser verschoben sein.

Wie insbesondere aus Fig. 10 ersichtlich ist, sind die Hebel 131, 132, 133, 134 sämtlich identisch ausgebildet. Auch die Lenker 141, 142, 143, 144 sind identisch ausgebildet; der Lenker 142 ist lediglich bezogen auf den Lenker 141 umgekehrt, das heißt um 180 Grad um eine Achse, die parallel zur Achse K1 ist, gedreht angeordnet. Auch die Stäbe 151, 152, 153, 154 sind identisch, wobei die Stäbe 153, 154 wiederum mit Bezug auf die Stäbe 151, 152 gedreht sind. Dieselben Lenkerelemente sind auch für die zweite Lenkeranordnung 112 verwendet. Dies ist nicht abgebildet, da die Abbildung identisch zu Abbildung 10 sein würde, und lediglich die Bezugszeichen angepasst wären.

In Fig. 10 sind weiterhin die ersten, zweiten, dritten und vierten Parallelogramme 91, 92, 93 und 94 mittels gestrichelter Linien eingezeichnet.

Ein weiteres Detail, das in Fig. 10 zu sehen ist, sind Gelenke 160 bis 169 der ersten Lenkeranordnung 110. Der erste Hebel 131 ist über ein erstes Gelenk 160 mit dem ersten Halter 141 gelenkig verbunden, und der zweite Hebel 132 ist über ein zweites Gelenk 161 mit dem ersten Halter gelenkig verbunden. Weiterhin ist der erste Stab 151 über ein drittes Gelenk 162 mit dem ersten Lenker 141 und über ein viertes Gelenk 163 mit dem ersten Halter 141 gelenkig verbunden. Der zweite Stab 152 ist über ein fünftes Gelenk mit dem ersten Halter 114 und auf der anderen Seite ebenfalls mittels des zweiten Gelenks 161 mit dem ersten Lenker 141 gelenkig verbunden. Das zweite Gelenk 161 bildet also ein gemeinsames Gelenk für die ersten und zweiten Parallelogramme 91, 92. Zwischen den ersten und zweiten Parallelogrammen 91, 92 ist ein Winkel β1 vorgesehen, der durch die Geometrie des ersten Lenkers 141 definiert wird, genauer gesagt durch die Anordnung der ersten, zweiten und dritten Gelenke 160 161, 162. Es hat sich herausgestellt, dass der Winkel β1 in einem Bereich von 90° bis <180° liegen soll, um die Verwendung von Gleichteilen, das heißt von identischen Stäben 151 bis 158, identischen Hebeln 131 bis 138 und identischen Lenkern 141 bis 144 zu ermöglichen.

Für das zweite Armsegment 82 gilt dasselbe wie für das erste Armsegment 80. Der dritte Hebel 133 ist über ein sechstes Gelenk mit dem zweiten Lenker 142, und der vierte Hebel 134 ist über ein siebtes Gelenk 166 mit dem zweiten Lenker 142 gelenkig verbunden. Der dritte Stab 153 ist wiederum über das siebte Gelenk 167 mit dem zweiten Lenker 142 und andererseits über ein zehntes Gelenk 169 mit dem ersten Halter 114 gelenkig verbunden. Der vierte Stab 154 ist über ein achtes Gelenk 167 mit dem zweiten Lenker 142 gelenkig, und über ein neuntes Gelenk 168 ist der vierte Stab 154 mit dem ersten Halter 114 gelenkig verbunden. Wiederum bilden die dritten und vierten Parallelogramme 93, 94 einen Winkel β2, der dem Winkel β1 entspricht.

Wie sich weiterhin aus Fig. 10 ergibt, sind die Hebelgelenkpunkte 121, 122, 123, 124 V-förmig angeordnet, und auch die Rotationsachsen R1, R2, R3, R4 sind V-förmig angeordnet. Die V-Form ist durch die beiden Achsen V1, V2 beschrieben, die in Fig. 10 als gestrichelte Linien eingezeichnet sind. Der Winkel α des V liegt in einem Bereich von >0° bis 90°. Die Spitze 150 des V, also der Schnittpunkt der Achsen V1, V2 liegt hier im ersten Halter 114 und damit im Arbeitsraum der Kinematik. Durch die V-förmige Anordnung der Hebelgelenkpunkte 121, 122, 123, 124, 125, 126, 127, 128 werden Singularitäten der ersten und zweiten Lenkeranordnung 110, 112 vermieden.

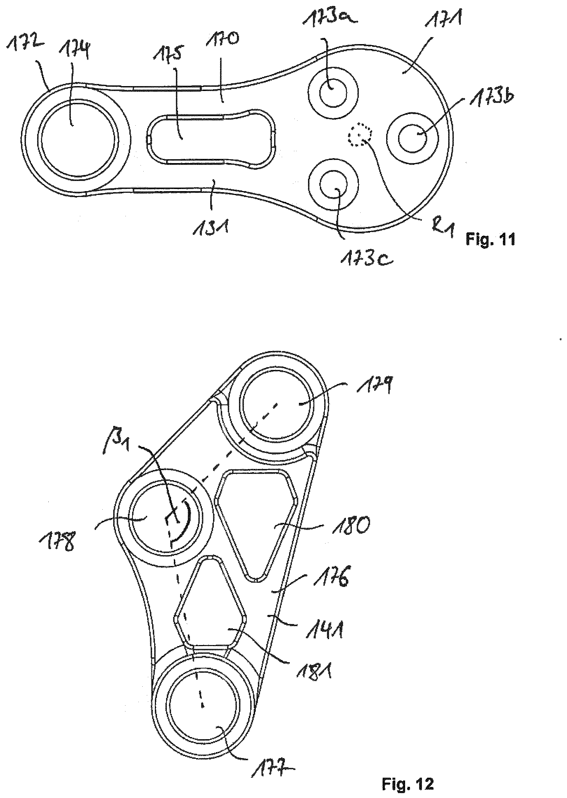

Die Fig. 11 bis 15 zeigen nun nochmals separat die drei Elemente (Hebel, Lenker und Stab) der Lenkeranordnungen 110, 112 sowie den Halter und das Kardanelement. Beispielhaft sind jeweils nur die ersten dieser Elemente gezeigt, wobei die weiteren Elemente immer identisch ausgebildet sind.

Der Lenker 131 (Fig. 11) ist aus einem Stück gefertigt und weist einen Rundkörper 170 auf mit einem Antriebsabschnitt 171 und einem Abtriebsabschnitt 172. Mit dem Antriebsabschnitt 171 ist der Hebel 131 gelenkig mit dem Rahmen verbunden und weist zur Befestigung drei Durchgangsöffnungen 173a, 173b, 173c auf, die zur Aufnahme von Befestigungsschrauben dienen. Die drei Durchgangsöffnungen 173a, 173b, 173c sind kreisförmig und äquidistant um die Rotationsachse R1 angeordnet. An dem Abtriebsabschnitt 172 ist eine weitere Durchgangsöffnung 174 ausgebildet, die zur Aufnahme des ersten Gelenks 160 dient. Im mittleren Abschnitt des Körpers 170 ist eine Öffnung 175 vorgesehen, die zu Gewichtsreduktionszwecken eingebracht ist. Die Hebel 132 bis 138 sind identisch ausgebildet.

Der Lenker 141 (vgl. Fig. 12) weist einen Grundkörper 176 auf. In den Grundkörper 176 sind eine erste Durchgangsöffnung 177 für das erste Gelenk 160, eine zweite Durchgangsöffnung 178 für das zweite Gelenk 161 und eine dritte Durchgangsöffnung 179 für das dritte Gelenk 162 ausgebildet. Diese Durchgangsöffnungen 177, 178, 179 sind jeweils so gestaltet, dass sie entsprechende Gelenkbuchsen der Gelenke 160, 161, 162 aufnehmen können. Die Durchgangsöffnungen 177, 178, 179 sind so angeordnet, dass sie den Winkel β1 einschließen, also den Winkel β1 zwischen den ersten und zweiten Parallelogrammen 91, 92. Durch entsprechende Gestaltung des ersten Halters 141 kann der Winkel β1 verändert werden. Der Winkel β1 hat beispielsweise auch Einfluss auf die Geometrie des ersten Halters 114, da der erste Halter 114 einen Teil des zweiten Parallelogramms 92 bildet.

Weiterhin weist der Körper 176 zwei weitere Durchgangsöffnungen 180, 181 auf, die wiederum aus Gewichtsgründen eingebracht sind. Wiederum sind die zweiten, dritten und vierten Lenker 142, 143, 144 identisch zu dem Lenker 141 ausgebildet.

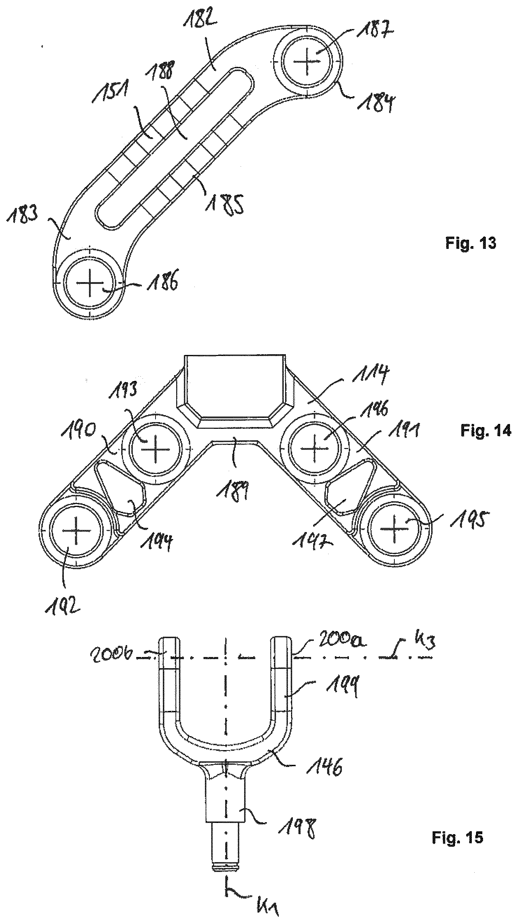

Der erste Stab 151 (Fig. 13) weist einen Grundkörper 182 auf. Zwei Endabschnitte 183, 184 erstrecken sich leicht gewinkelt jeweils in etwa unter einem 45-Grad-Winkel von dem Mittelteil 185 des Stabs 151. In den Endabschnitten 183, 184 sind Durchgangsöffnungen 186, 187 eingebracht, zur Aufnahme des dritten Gelenks 162 und vierten Gelenks 163. Wiederum sind die Durchgangsöffnungen 186, 187 so gebildet, dass Lagerbuchsen aufgenommen werden können. In dem Mittelteil 185 ist eine längliche Durchgangsöffnung 188 eingebracht, die wiederum im Wesentlichen aus Gewichtsgründen vorgesehen ist. Allerdings bietet die Durchgangsöffnung 188 auch ein verbessertes Sichtfeld für den Operateur oder Bediener, wenn dieser die chirurgische Manipulatorvorrichtung 100 gemäß der Erfindung verwendet. Die weiteren Stäbe 152 bis 158 sind identisch zum ersten Stab 151 gebildet. Der Stab 151 ist insgesamt einstückig ausgebildet und beispielsweise aus Aluminium gefräst.

Der erste Halter 114 (vgl. Fig. 14) weist einen Grundkörper 189 auf und ist insgesamt einstückig hergestellt. Der Halter 114 weist einen ersten und einen zweiten Arm 190, 191 auf, wobei der erste Arm 190 für den ersten und zweiten Stab 151, 152 und der zweite Arm 191 für die dritten und vierten Stäbe 153, 154 vorgesehen ist. Zu diesem Zweck sind in dem ersten Arm 190 eine erste und zweite Durchgangsöffnung 192, 193 eingebracht, die zur Aufnahme von Gelenkbuchsen dienen und eine Aufnahme der vierten und fünften Gelenke 163, 164 bilden. Zwischen diesen ersten und zweiten Durchgangsöffnungen 192, 193 ist eine dritte Durchgangsöffnung 194 aus Gewichtsreduktionszwecken angebracht. In entsprechender Weise weist der zweite Arm 191 ein erstes und zweites Durchgangsloch 195, 196 auf, die zur Aufnahme von Gelenkbuchsen ausgebildet sind und dazu dienen, die neunten und zehnten Gelenke 168, 169 aufzunehmen. Zwischen diesen ist wiederum eine dritte Durchgangsöffnung 197 vorgesehen, die aus Gewichtsreduktionszwecken eingebracht ist. Der zweite Halter 116 ist identisch gebildet.

Das erste Kardanelement 146 (vgl. Fig. 15) ist einstückig beispielsweise aus Aluminium gebildet. Es weist einen Schaft 198 auf, der in der Aufnahme 148 aufgenommen werden kann. Von dem Schaft 198 erstreckt sich eine Gabel 199, die zur Aufnahme der Instrumentenaufnahmeeinrichtung 120 dient. Die Gabel 199 weist dazu (in Fig. 15 nicht sichtbare) Durchbrüche 200a, 200b auf, entlang einer Achse K3, die senkrecht zur Achse K1 ist. Auf diese Weise lässt sich die Instrumentenaufnahmeeinrichtung 120 vollständig rotierbar an den ersten und zweiten Haltern 114, 116 aufnehmen.

In einer Ausführungsform der chirurgischen Manipulatorvorrichtung 100 weist diese einen Antrieb 210 auf (Fig. 16). In Fig. 16 ist das Gehäuse 104 und auch Platinen, die innerhalb des Gehäuses 104 angeordnet sind, weggelassen, um den Antrieb 210 sichtbar zu machen. Der Antrieb 210 umfasst in diesem Ausführungsbeispiel vier Motoren 211, 212, 213, 214. Von diesen vier Motoren 211, 212, 213, 214 sind die Motoren 212, 214 in Fig. 16 verdeckt durch die Motoren 211, 213. Während der erste und zweite Motor 211, 212 für die erste Lenkeranordnung 110 vorgesehen ist, sind die dritten und vierten Motoren 213, 214 für die zweite Lenkeranordnung 112 vorgesehen. Die Motoren 211, 212, 213, 214 sind mit ihren Drehachsen auf den Rotationsachsen R1, R2, R3, R4 angeordnet. Das heißt, der erste Motor 211 ist mit dem ersten Hebel 131 gekoppelt, und der zweite Motor 212 ist mit dem dritten Hebel 133 gekoppelt. In entsprechender Weise ist der dritte Motor 213 mit dem sechsten Hebel 136 und der vierte Motor 214 mit dem achten Hebel 138 gekoppelt. Die ersten und zweiten Motoren 211, 212 treiben also die distal zum ersten Halter 114 angeordneten Hebel 131, 133 an, während die dritten und vierten Motoren 213, 214 die proximal zum zweiten Halter 116 angeordneten Hebel 136, 138 antreiben. Vorzugsweise ist zwischen den Motoren 211, 212, 213, 214 und den entsprechenden Hebeln 131, 133, 136, 138 ein Getriebe zur Drehmoment- und Drehzahlwandlung vorgesehen. Die Motoren 211, 212, 213, 214 sind hier als bürstenlose Gleichstrommotoren ausgebildet.

Aufgrund der besonderen Doppel-Parallelkinematik der ersten und zweiten Lenkeranordnungen 110, 112 reicht es aus, dass jeweils nur zwei Hebel der vier Hebel Lenkeranordnung 110, 112 angetrieben werden. Hierdurch können auch die Motoren 211, 212, 213, 214 so platziert werden, dass diese benachbart zueinander sind, parallel nebeneinander und nicht auf einer gemeinsamen Rotationsachse axial versetzt. Hierdurch kann die Baugröße der chirurgischen Manipulatorvorrichtung 100 deutlich reduziert werden, wie insbesondere aus Fig. 16 ersichtlich.

Zur Steuerung der Motoren 211, 212, 213, 214 ist eine Steuereinheit vorgesehen, die über die Schnittstelle 106 Signale empfängt. Dies wird weiter unten noch im Detail beschrieben werden.

In Fig. 17 ist eine zweite Ausführungsform der Manipulatorvorrichtung 100 gezeigt, die keinen Antrieb 210, sondern eine Bremseinrichtung 220 zum aktiven Bremsen der ersten und zweiten Lenkeranordnungen 110, 112 aufweist. Die Bremseinrichtung 220 weist vier Bremsen 221, 222, 223, 224 auf, die auf ähnliche Weise an dem Rahmen 108 befestigt sind, wie die Motoren 211, 212, 213, 214. Wiederum verdeckt die erste Bremse 221 die zweite Bremse 222, und die dritte Bremse 223 verdeckt die vierte Bremse 224. Die Bremsen 221, 222, 223, 224 sind jeweils koaxial zu den Rotationsachsen R1, R2, R3, R4 angeordnet. Die erste Bremse 221 ist mit dem ersten Hebel 131 gekoppelt, die zweite Bremse 222 entsprechend mit dem dritten Hebel 133. In entsprechender Weise ist die dritte Bremse 223 mit dem sechsten Hebel 136 und die vierte Bremse 224 mit dem achten Hebel 138 gekoppelt. Die Bremsen 221 bis 224 sind als elektromagnetische Bremsen ausgebildet, die im stromlosen Zustand zugespannt sind.

Zum Freigeben der Bremseinrichtung weist die chirurgische Manipulatorvorrichtung 100 eine Freigabeeinheit 225 auf. Mittels der Freigabeeinheit 225 können eine oder mehrere Freiheitsgrade der ersten und/oder zweiten Lenkeranordnungen 110, 112 freigegeben werden. In diesem Ausführungsbeispiel (Fig. 17) ist die Freigabeeinheit 225 als Taster 226 ausgebildet und ebenfalls an dem Rahmen 108 angeordnet, und zwar so, dass er außerhalb des Gehäuses 204 zugänglich ist (vgl. insbesondere Fig. 23). Durch Drücken dieses Tasters 226 werden sämtliche Bremsen 221, 222, 223, 224 freigegeben, und die ersten und zweiten Lenkeranordnungen 110, 112 und so auch die Position der ersten und zweiten Halter 114, 116 kann verstellt werden. Auf diese Weise ist eine passive chirurgische Manipulatorvorrichtung gebildet, die von einem Bediener auf einfache Art und Weise, nämlich durch Drücken des Tasters 226 und manuelles Verstellen in eine gewünschte Pose verbracht werden kann und dort arretiert wird. Zum Arretieren ist lediglich der Taster 226 wieder loszulassen. Sodann werden die Bremsen 221, 222, 223, 224 stromlos geschaltet und spannen sich zu. Die Pose der ersten und zweiten Lenkeranordnungen 110, 112 ist arretiert.

Wie bereits oben erwähnt wurde, kann an den ersten und zweiten Haltern 114, 116, gegebenenfalls unter Zwischenschaltung der ersten und zweiten Kardanelemente 146, 147 eine Instrumentenaufnahmeeinrichtung 120 aufgenommen werden. Die Instrumentenaufnahmeeinrichtung, wie in den Figuren 18 und 19 gezeigt, wird zudem unabhängig von der Manipulatorvorrichtung offenbart. Die Instrumentenaufnahmeeinrichtung 120 kann auch mit anderen Manipulatoren verwendet werden und nicht zwangsläufig mit der chirurgischen Manipulatorvorrichtung, die in den Figuren 1 bis 17 erläutert wurde.

Die in dem Ausführungsbeispiel gemäß Fig. 18 und 19 dargestellte Instrumentenaufnahmeeinrichtung 120 ist nicht nur als starres Stativ verwendbar (obwohl auch dies im Rahmen der Erfindung bevorzugt ist), sondern weist einen Linearantrieb 230 auf. Im Detail weist die Instrumentenaufnahmeeinrichtung 120 zunächst ein Fußgehäuse 232 auf, welches an seiner Außenseite eine Vertiefung 233 hat (mit Bezug auf Fig. 18 ist an der gegenüberliegenden Seite des Fußgehäuses 232 ist eine weitere Ausnehmung angeordnet), mittels derer das Fußgehäuse 232 formschlüssig, aber schwenkbar um die Achse K3 in einem Kardanelement 146, 147, insbesondere dem unteren Kardanelement 147, aufgenommen werden kann (vgl. auch Fig. 2 und 15). Von dem Fußgehäuse 232 erstreckt sich in Bezug auf Fig. 18 nach oben eine Hülse 234, die fest mit dem Fußgehäuse 232 verbunden ist, entlang einer Längsachse L1. Die Hülse 234 hat eine im Wesentlichen rechteckige oder quadratische Grundform (vgl. auch Fig. 1) und ist vorzugsweise aus einem Kunststoff oder einem nicht-magnetischen Material gebildet.

Ein zweiter Befestigungspunkt für die Instrumentenaufnahmeeinrichtung 120 ist durch eine Rutschkupplung 235 gebildet, die verschieblich entlang der Längsachse L1 an einer Außenseite der Hülse 234 gelagert ist, wie durch die gestrichelten Linien der Rutschkupplung 235' in Fig. 18 angedeutet. Die Rutschkupplung 235 weist ebenfalls zwei gegenüberliegende Ausnehmungen 236 auf, wie schon mit Bezug auf das Fußgehäuse 232 beschrieben wurde. Über die Ausnehmung 236 ist die Hülse 235 formschlüssig, aber drehbar mit dem ersten Kardanelement 146 verbunden (vgl. Fig. 2). Die Rutschkupplung 235 dient dazu, einen sich ändernden Abstand zwischen den ersten Ausnehmungen 233 und den zweiten Ausnehmungen 236 auszugleichen, wenn die ersten und zweiten Lenkeranordnungen 110, 112 nicht in Übereinstimmung miteinander verschwenkt werden. So ist beispielsweise denkbar, dass die erste Lenkeranordnung 110 in eine Position wie in Fig. 6 gezeigt verschwenkt wird, und die zweite Lenkeranordnung 112 in eine Position wie in Fig. 7 gezeigt verschwenkt wird. In diesem Fall würde sich die Längsachse L1 nicht senkrecht auf die Bewegungsebenen B1, B2 erstrecken, sondern schräg zu diesen. In diesem Fall wäre der Abstand zwischen den Ausnehmungen 236, 233 relativ zu der Situation, wenn die Längsachse L1 senkrecht auf die Bewegungsebenen B1, B2 steht, vergrößert. Um diesen Abstand auszugleichen, ohne Spannungen oder Verformungen in die ersten und zweiten Lenkeranordnungen 110, 112 einzubringen, ist die Rutschkupplung 235 verschieblich angeordnet.

Die Rutschkupplung 235 hat vorzugsweise einen möglichst engen Schluss mit der Hülse 234, ohne aber eine zu hohe Reibung zu entwickeln. Hierzu kann die Rutschkupplung 235 auf einer Innenseite mit entsprechenden Materialien versehen sein.

Der Linearantrieb 230 weist in dieser Ausführungsform einen in der Hülse 234 angeordneten Spindeltrieb 238 auf. Der Spindeltrieb 238 umfasst eine Spindel 239, die sich entlang der Längsachse L1 im Inneren der Hülse 234 erstreckt. Am mit Bezug auf Figur 19 oberen axialen Ende 240 ist die Spindel 239 mit einem Drehlager 241 aufgenommen. Am mit Bezug auf Figur 19 unteren Ende 242 ist die Spindel 239 mit einem Elektromotor 243 gekoppelt, der die Spindel 239 um die Längsachse L1 rotierbar antreibt. In einem Fortsatz 244 des Fußgehäuses 232 ist eine entsprechende Steuereinheit 245 für den Elektromotor 243 sowie eine Schnittstelle 246 zum Übertragen von Signalen an die Steuereinheit 245 angeordnet.

Auf der Spindel 239 ist ein Magnetmitnehmer 250 angeordnet, der über ein Innengewinde 251 mit dem Außengewinde der Spindel 239 in Eingriff steht. Durch Rotation der Spindel 239 lässt sich der Magnetmitnehmer 250 entlang mit Längsachse L1 bewegen. Der Magnetmitnehmer 250 weist an seiner radial äußeren Seite eine Mehrzahl Permanentmagneten 252 auf. Der Linearantrieb 230 weist darüber hinaus ein Abtriebselement 254 in Form einer Buchse auf, die an der Außenseite der Hülse 234 gleitbar entlang der Längsachse an dieser angeordnet ist. Auf der Innenseite trägt das Abtriebselement 254 eine weitere Anzahl Permanentmagneten 255, die mit den Permanentmagneten 252 korrespondieren. Auf diese Weise ist das Abtriebselement 254 mit dem Magnetmitnehmer 250 gekoppelt und somit ist das Abtriebselement 254 an mittels Rotation der Spindel 239 entlang der Längsachse L1 hin- und herbewegbar. Auf diese Weise kann die Hülse 234 vollständig geschlossen ausgebildet sein und benötigt keinerlei Vertiefungen oder Vorsprünge an der Außenseite, wodurch die Hygiene der chirurgischen Manipulatorvorrichtung 100 der vorliegenden Erfindung wesentlich verbessert ist.

Das Abtriebselement 254 weist ferner ein erstes Ankopplungselement 256 auf, das in diesem Fall als hakenförmiger Haltefinger ausgebildet ist. Das erste Ankopplungselement 256 ist über eine Schraubverbindung 257 mit dem Buchsenkörper des Abtriebselements 254 verbunden. Das erste Ankopplungselement 256 ist vorzugsweise aus einem isolierenden Kunststoff gebildet, der ein über Formschlussmittel 258 aufgenommenes chirurgisches Instrument gegenüber dem Linearantrieb 230 und vorzugsweise gegenüber dem Buchsenkörper des Abtriebselements 254 isoliert.

Für den Fall, dass die Instrumentenaufnahmeeinrichtung 120 eine Kraft-Momenten-Sensoreinheit 260 aufweist, ist deren Anbringung an dem ersten Ankopplungselement 256 bevorzugt. Sämtliche Kräfte, die von einem chirurgischen Instrument 102 auf die chirurgische Manipulatorvorrichtung 100 wirken, werden über das erste Ankopplungselement 256 geleitet. Die Kraft-Momenten-Sensoreinheit 260 kann beispielsweise einen Kraft-Momenten-Sensor aufweisen, der zwischen die Schraubverbindung 257 angeordnet ist. Ferner ist es auch denkbar, dass einzelne Kraftsensoren, wie beispielsweise Dehnmessstreifen, direkt auf einer Oberfläche des ersten Ankopplungselements 256 angeordnet sind. Die Kraft-Momenten-Sensoreinheit 260 ist vorzugsweise mit der Steuereinheit 245 verbunden und/oder stellt Signale über die Schnittstelle 246 bereit.

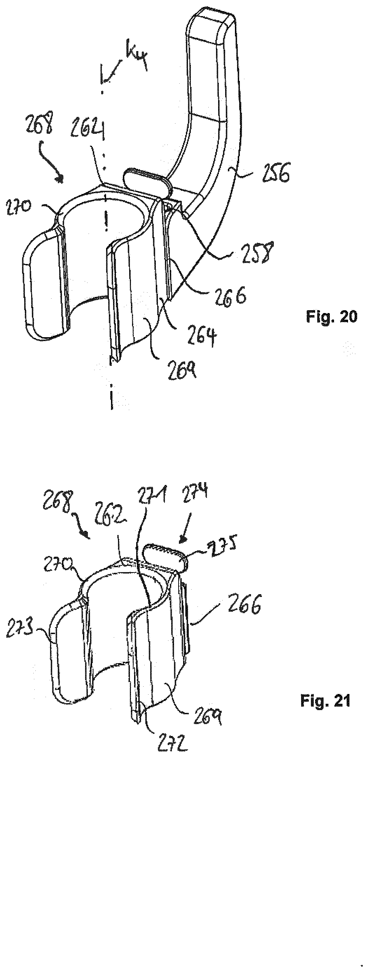

Das erste Ankopplungselement 256 ist in perspektivischer Darstellung in Figur 20 nochmals gezeigt. In der in Figur 20 gezeigten Darstellung trägt das erste Ankopplungselement 256 ein zweites Ankopplungselement 262, welches zur klemmenden Aufnahme eines chirurgischen Instruments vorgesehen ist.

Das Ankopplungselement 262 weist einen Hauptkörper 264 aus einem flexiblen elektrisch isolierenden Material auf, der Formschlussmittel 266 zur Kopplung mit den Formschlussmitteln 258 der Instrumentenaufnahmeeinrichtung 120 aufweist. Gegenüberliegend zu den Formschlussmitteln 266 bildet der Grundkörper 264 einen Klemmabschnitt 268. Der Klemmabschnitt 268 weist eine erste und eine zweite Klemmbacke 269, 270 auf, die spiegelsymmetrisch zueinander ausgebildet sind. Die Klemmbacken 269 umschließen teilkreisförmig eine Zentralachse K4. Der teilkreisförmige Abschnitt der Klemmbacken 269, 270 entspricht in etwa einem Dreiviertelkreis. Die Klemmbacken 269, 270 weisen am Ende des kreisförmigen Abschnitts 271 jeweils eine Lasche 272, 273 auf, die axial dieselbe Länge wie die Klemmbacken 269, 270 aufweisen. Die Laschen 272, 273 weiten sich auf, indem sie ausgehend von der Achse K4 voneinander weg verlaufen. Ebenen, die durch die Laschen 272, 273 gebildet werden, schneiden sich vorzugsweise in der Zentralachse K4. Die Laschen 272, 273 dienen einerseits durch ihre Anschrägung dem leichten Einführen des Instruments in den Raum zwischen den Klemmbacken 269, 270, andererseits auch dem leichten Entfernen des Instruments 102 durch manuelles Ergreifen der Laschen 272, 273 mit den Händen und Auseinaderdrücken der Klemmbacken 269, 270.

Die Klemmbacken 269, 270 bieten eine formschlüssige Fixierung des chirurgischen Instruments 102 in Richtungen senkrecht zur Zentralachse K4, erlauben aber ein Verschieben des chirurgischen Instruments 102 in Richtung der Zentralachse K4, wie dies etwa in Figur 2 gesehen werden kann.

Im Bereich der Formschlussmittel 266 weist das Ankopplungselement 262 ferner eine Verclipseinrichtung 274 mit einem Rastfinger und einer Rastnase auf, die in eine entsprechende Ausnehmung am ersten Ankopplungselement 262 eingreifen kann. Durch den Griff 275 lässt sich die Rastnase aus der entsprechenden Rastnut am ersten Ankopplungselement 256 herausheben und so die formschlüssige Verbindung zwischen dem zweiten Ankopplungselement 262 und dem ersten Ankopplungselement 256 lösen. Die Formschlussmittel 266, 258 können beispielsweise als Schwalbenschwanzführung oder dergleichen ausgebildet sein.

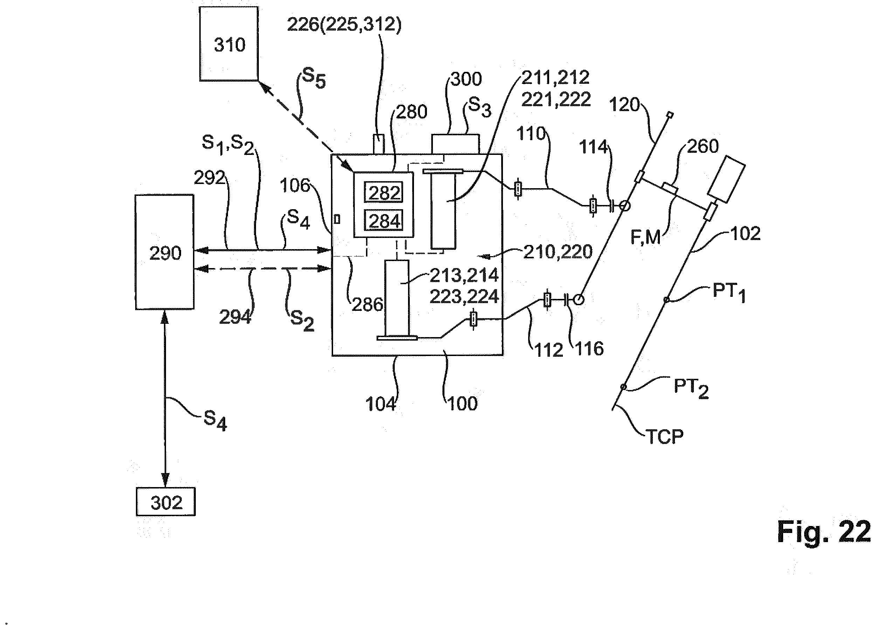

In der schematischen Darstellung (Fig. 22) ist die chirurgische Manipulatorvorrichtung 100 mit weiteren Peripheriegeräten dargestellt. Die chirurgische Manipulatorvorrichtung 100 weist wiederum die ersten und zweiten Lenkeranordnungen 110, 112 auf, die in der Figur 22 vereinfacht dargestellt sind. In einer ersten Variante weist die Manipulatorvorrichtung 100 einen Antrieb 210 und entsprechende erste, zweite, dritte, vierte Motoren 211, 212, 213, 214 auf. In einer zweiten, ebenfalls in Figur 22 gezeigten Variante, weist die Manipulatorvorrichtung 100 eine Bremseinrichtung 220 mit entsprechenden ersten, zweiten, dritten und vierten Bremsen 221, 222, 223, 224. Da sich die schematische Konstruktion betreffend den Antrieb 210 und die Bremseinrichtung 210, nicht unterscheidet, können diese in anderen Figuren dargestellt werden.

Figur 22 soll insbesondere die elektronische Steuereinheit 280 illustrieren. Die elektronische Steuereinheit280 weist Speichermittel 282 und einen Prozessor 284 zum Steuern der Bewegung und Positionierung der ersten und zweiten Halter 114,116 auf. Die elektronische Steuereinheit 280 ist über eine Leitung 286 mit der Schnittstelle 106 verbunden, die als mechatronische Schnittstelle ausgebildet ist. Über diese Schnittstelle 106 ist die chirurgische Manipulatorvorrichtung mit einer übergeordneten Steuereinheit 290 verbunden. Diese übergeordnete Steuereinheit 290 kann ein OP-Navigationssystem oder auch der chirurgische Haltearm 1 sein. Die Verbindung zwischen der übergeordneten Steuereinheit 290 und der Manipulatorvorrichtung 100 kann physisch sein, über eine Leitung oder ein Kontakt 292 an der elektronischen Schnittstelle 106, oder drahtlos über eine Funkverbindung 394. Beispielsweise können die übergeordnete Steuereinheit 290 und die Manipulatorvorrichtung 100 mittels Infrarotstrahlung kommunizieren und die mechatronische Schnittstelle 106 ist in diesem Fall mit einem entsprechenden Empfänger ausgestattet.

Von der übergeordneten Steuereinheit 290 kann die Steuereinheit 280 der Manipulatorvorrichtung 100 beispielsweise direkt Stellsignale S1 für den Antrieb 210 und/oder die Bremseinrichtung 220 empfangen. Für diese Ausführungsformen benötigt die Steuereinheit 280 keine besondere Intelligenz, sondern muss lediglich entsprechende Stellsignale an den Motoren 211, 212, 213, 214, bzw. den Bremsen 221, 222, 223, 224 bereitstellen. Es kann aber auch vorgesehen sein, dass von der übergeordneten Steuereinheit 290 Positionsanforderungssignale S2 bereitgestellt werden, das heißt, beispielsweise eine Position, an der ein Tool Center Point, bzw. Spitze des chirurgischen Instruments 102, oder ein Pivotpunkt PT1, PT2 des chirurgischen Instruments 102 liegen soll. Für diesen Fall sind in den Speichermitteln 282 Daten hinterlegt, die die ersten und zweiten Lenkeranordnungen 110, 112 repräsentieren, bzw. die vollständige Kinematik von im übergeordneten Koordinatensystem, beispielsweise dem Koordinatensystem eines Navigationssystems, bis zum entsprechenden Tool Center Point TCP oder Pivotpunkt PT1, PT2.

Ferner sind in der Speichereinheit 282 Softwaremittel gespeichert, die, wenn durch den Prozessor 284 ausgeführt, folgende Schritte ausführen: Ermitteln eines ersten Vektors und/oder Trajektorie für einen ersten Halter 214, Ermitteln eines zweiten Vektors und/oder einer zweiten Trajektorie für den zweiten Halter 216, Bereitstellen von Stellsignalen an die Motoren 111, 112, 113, 114 zum Bewegen des ersten Halters 14 in Übereinstimmung mit dem ersten Vektor bzw. der ersten Trajektorie und zum Bewegen des zweiten Halters 16 in Übereinstimmung mit dem zweiten Vektor, bzw. der zweiten Trajektorie. Vorzugsweise wird in der Regel eine Trajektorie bestimmt, da bei der Bewegung des Instruments 102 nicht nur der Zielpunkt von Bedeutung ist, sondern auch der Weg von einer Ist-Position zu einer Soll-Position berücksichtigt werden muss. Auf diesem Weg kann es vorkommen, dass das chirurgische Instrument 102 mit Körperteilen des Patienten kollidiert, und aus diesem Grund eine bestimmte Trajektorie gewählt werden muss, um eine Kollision zu vermeiden.

Für den Fall, dass eine Kraft-Momenten-Sensoreinheit 260 vorgesehen ist, ist auch diese mit der Steuereinheit 280 verbunden. Die Verbindung kann kabelgebunden oder drahtlos sein. Die Steuereinheit 280 weist vorzugsweise in der Speichereinheit 282 entsprechende Softwaremittel auf, die dazu eingerichtet sind, die von der Kraft-Momenten-Sensoreinheit 260 bereitgestellten Signale zu verarbeiten und den Antrieb 210 und/oder einen Linearantrieb der Instrumentenaufnahmeeinrichtung 120, oder auch gegebenenfalls die Bremseinheit 220 entsprechend zu steuern. Die Kraft, die von dem chirurgischen Instrument 102 auf die Instrumentenaufnahmeeinrichtung 120 wirkt, kann einen Bedienerwunsch darstellen. So ist es beispielsweise möglich, dass ein Operateur das chirurgische Instrument 102 manuell ergreift und zu einem bestimmten Punkt führen will. In diesem Fall wirken von dem chirurgischen Instrument 102 auf die Instrumentenaufnahmeeinrichtung 120 Kräfte F und Momente M, die mittels der Kraft-Momenten-Sensoreinheit erfasst werden. entsprechende Signale werden dann an der Steuereinheit 280 bereitgestellt. Die Softwaremittel sind vorzugsweise ausgebildet, wenn auf dem Prozessor 284 ausgeführt, die Steuereinheit 280 zu veranlassen, Bewegung, bzw. Trajektorie oder Vektor zu ermitteln für die ersten und zweiten Lenkeranordnungen 110, 112 und/oder einen Linearantrieb 238 der Instrumentenaufnahmeeinrichtung 120, um die auf die Instrumentenaufnahmeeinrichtung 120 wirkenden Kräfte F und Momente M auszugleichen sowie Bereitstellen von Steuersignalen an den Antrieb 210, den Linearantrieb 238 und/oder die Bremseinrichtung 220 in Übereinstimmung mit dieser Bewegung, Trajektorie oder Vektor, um die Bewegung entsprechend der Trajektorie oder dem Vektor auszuführen. Das heißt, der chirurgische Manipulator 100 gibt dem Bedienerwunsch nach und versucht, eine Pose einzunehmen, die die auf die Instrumentenaufnahmeeinrichtung 120 wirkenden Kräfte F und Momente M ausgleichen.

Da nicht zu jedem Zeitpunkt ein solches Vorgehen gewünscht ist, ist es bevorzugt, dass der chirurgische Manipulator 100 ein integriertes Eingabesystem 300 aufweist. Das integrierte Eingabesystem 300 kann beispielsweise ein Mikrofon umfassen und/oder ein Touchdisplay, um die chirurgischen Manipulatorvorrichtung 100 in diesen Modus zu versetzen, indem die beschriebene Software ausgeführt wird. Beispielsweise kann vorgesehen sein, dass der Bediener ein Kommando "manueller Führungsmodus" oder "Nachfolgemodus" eingeben muss und dieses Sprachkommando mittels des Mikrofons erfasst und entsprechende Signale an der Steuereinheit 280 bereitgestellt werden. Durch entsprechende Spracherkennungssoftwaremittel werden Stellsignale bereitgestellt, sodass die Softwaremittel auf dem Prozessor 284 ausgeführt werden.

Alternativ oder zusätzlich kann auch ein Fußpedal 302 vorgesehen sein, welches in diesem Ausführungsbeispiel mit der übergeordneten Steuereinheit 290 gekoppelt ist. Über das Fußpedal 302 kann ein entsprechendes Signal S4 an die übergeordnete Steuereinheit 290 bereitgestellt werden, sodass die übergeordnete Steuereinheit 290 das entsprechende Signal S4 weiterleitet und an der Steuereinheit 280 der Manipulatorvorrichtung 100 bereitstellt.

In einer weiteren Ausführungsform kann vorgesehen sein, dass die chirurgische Manipulatorvorrichtung 100 mit einem handgehaltenen Mobilgerät 310 kommuniziert. Das Mobilgerät 310 kann beispielsweise ein Tablet-PC, ein Mobiltelefon oder ein anderes nach Art einer Fernbedienung gebildetes Gerät sein. Von dem Mobilgerät 310 können Signale S5 drahtlos an die Steuereinheit 280 übertragen werden, die dazu einen entsprechenden Empfänger aufweist. Das Mobilgerät 310 kann auch dazu ausgebildet sein, eine Repräsentation der Pose der Manipulatorvorrichtung 100 anzuzeigen, beispielsweise eine grafische vereinfachte Darstellung der Pose. Es kann vorgesehen sein, dass auf diesem Mobilgerät eine Software ausgeführt wird, die eine Positionierung des chirurgischen Instruments mittels Drag & Drop, insbesondere über ein Touchdisplay erlaubt. Ist auf dem Mobilgerät beispielsweise auch der Patient mit bestimmten Landmarken versehen abgebildet, kann durch Auswählen einer bestimmten Landmarke eine Positionierung des chirurgischen Instruments über das Mobilgerät 310 automatisiert erfolgen. Die Verarbeitung der Positionsanforderungssignale, die entsprechend als Signal S5 an die Steuereinheit 280 gesendet werden, erfolgt in oben beschriebener Weise.

Auf ähnliche Weise kann auch das integrierte Eingabesystem 300 ein Display aufweisen, auf dem solche Repräsentationen angezeigt werden.

Selbstverständlich ist es auch möglich, über das integrierte Eingabesystem 300, oder das Mobilgerät 310, Warnsignale auszugeben, wenn beispielsweise ein vordefinierter Arbeitsraum der chirurgischen Manipulatorvorrichtung 100 verlassen wird, beispielsweise wenn ein Bediener in dem manuellen Positionierungsmodus, wie oben beschrieben, manuell das chirurgische Instrument 102 positioniert und dabei festgestellt wird, dass dieses aus einem vordefinierten Arbeitsraum herausgeführt wird.

An dem Gehäuse 104 ist ein Taster 226 angeordnet. Der Taster 226 wurde oben bereits mit Bezug auf die Bremseinrichtung 220 beschrieben. Der Taster 226 kann je nach Ausgestaltung der chirurgischen Manipulatorvorrichtung, also ob ein Antrieb 210 oder eine Bremseinrichtung 220 vorgesehen ist, belegt werden. Ist die Bremseinrichtung 220 vorgesehen, ist der Taster 226 vorzugsweise als Freigabeeinrichtung 225 parametrisiert und über den Taster 226 können die Bremsen 221, 222, 223, 224 freigegeben werden.

Für den Fall, dass anstelle der Bremseinrichtung 220 ein Antrieb 210 mit Motoren 211, 212, 213, 214 vorgesehen ist, ist der Taster 226 vorzugsweise als sogenannter Home-Button 312 parametrisiert. Durch Betätigen des Tasters 226 wird in dieser Ausführungsform, wenn er als Home-Button 312 parametrisiert ist, eine Ausgangspose der chirurgischen Manipulatorvorrichtung, die vordefiniert und in dem Speicher 282 vorgespeichert ist, angefahren. Auf diese Weise ist es möglich, eine vorbestimmte und vorgespeicherte Pose durch einfaches Drücken des Tasters 226 anzufahren. Vorzugsweise ist ferner vorgesehen, dass die vorgespeicherte Pose mittels des Tasters 226 abgespeichert werden kann. Hierzu wird der Taster 226 für eine vorbestimmte Zeitdauer (beispielsweise 3 Sekunden) gedrückt gehalten, und die Ist-Pose wir als vorgespeicherte Pose in den Speichermitteln 282 hinterlegt, um durch erneutes Drücken des Tasters 226 aufgerufen und angefahren zu werden.

Um einen Pivotpunkt für ein chirurgisches Instrument 102 initial einzulesen, oder festzulegen, kann ein gemäß der vorliegenden Erfindung als chirurgisches Instrument 102 eine Pivopunktlehre 320 an den ersten und zweiten Halter 114, 116 aufgenommen werden. Dies ist in Figur 23 dargestellt. Die Pivotpunktlehre 320 ist genauer gesagt an der Instrumentenaufnahmeeinrichtung 120 und dort mittels des zweiten Ankopplungselements 262, welches mit Bezug auf Figur 21 im Detail beschrieben wurde, aufgenommen.

Die Pivotpunktlehre 320 weist einen Schaft 322 und einen verjüngten Bereich 324 auf. Der verjüngte Bereich ist so ausgebildet, dass er zwischen den Klemmbacken 270, 269 aufgenommen werden kann. Die axiale Erstreckung des verjüngten Abschnitts 224 entspricht dem der axialen Länge der Klemmbacken 269, 270 mit Bezug auf die Achse K4. Das heißt, die Pivotpunktlehre 320 hat eine definierte Position bezüglich des Ankopplungselements 262 und somit auch bezüglich eines Koordinatensystems der chirurgischen Manipulatorvorrichtung 100. Die axiale Länge der Pivotpunktlehre 320 ist bekannt und somit auch die Position des Tastkopfes 324, der an einem axialen Ende der Pivotpunktlehre 320 ausgebildet ist. Zum Einlesen eines Pivotpunkts PT führt nun der Bediener manuell, oder elektrisch gesteuert, die Manipulatorvorrichtung 100 in eine Pose, in der der Tastkopf 324 an dem patientenspezifischen Pivotpunkt des Patienten angeordnet ist.

Die Steuereinheit 280 weist für diesen Fall vorzugsweise Softwaremittel auf, die, wenn auf dem Prozessor 284 ausgeführt, über die Stellung der einzelnen Gelenke und die Pose der ersten und zweiten Lenkeranordnungen 110, 112 sowie die Position und Stellung der Instrumentenaufnahmeeinrichtung 120, Koordinaten des Tastkopfes 324 und somit auch des Pivotpunkts PT berechnen. Zum Abspeichern dieser Pivotpunktkoordinaten, und/oder zum Bereitstellen der Pivotpunktkoordinaten an der Schnittstelle 106, tätigt ein Bediener vorzugsweise eine Benutzereingabe, beispielsweise betätigt er den Taster 226, der hierfür parametrisiert sein kann. Es kann auch vorgesehen sein, dass an der Pivotpunktlehre 320 selbst ein Taster oder dergleichen vorgesehen ist, um das Abspeichern und/oder Bereitstellen der Koordinaten durchzuführen.

Die Steuereinheit 280 ist vorzugsweise ferner dazu eingerichtet, bei Aufnahme eines anderen chirurgischen Instruments 102, das nicht dieselbe axiale Länge wie der Schaft 322 der Pivotpunktlehre 320 hat, bezogen auf dieses chirurgische Instrument, den Pivotpunkt zu berechnen, und entsprechend axial entlang der Längsachse K4 zu verschieben. Ist beispielsweise ein Endoskop aufgenommen (vgl. Fig. 2), kann die axial untere Spitze des Endoskops abweichend von dem Tastkopf 324 positioniert sein. Wenn die Geometrie des Endoskops bekannt ist, kann die Position des Pivotpunkts entsprechend transformiert werden.

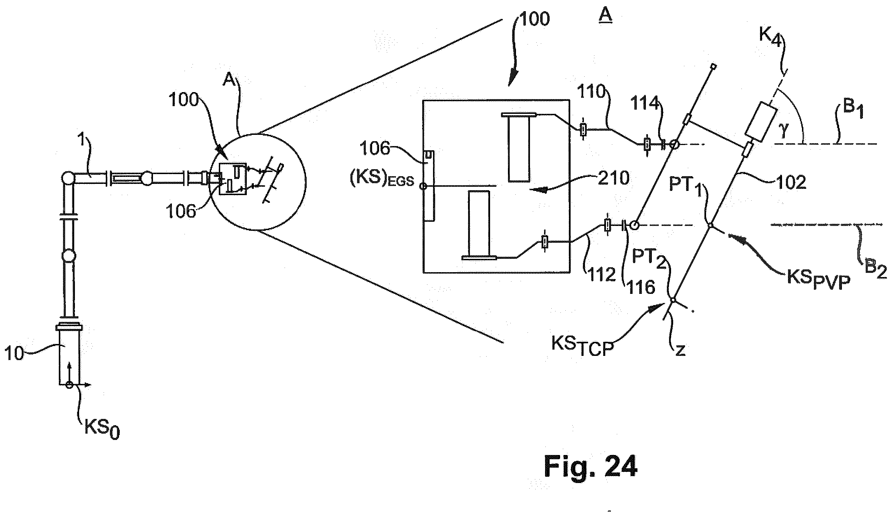

Figur 24 illustriert nochmals die Bestimmung des Pivotpunkts PT und die entsprechende Berechnung. In Figur 24 ist die chirurgische Manipulatorvorrichtung 100 wiederum schematisch dargestellt, während das Detail A aus der linken Seite der Figur 24 rechts nochmals vergrößert dargestellt ist. Die chirurgische Manipulatorvorrichtung 100 ist in diesem Ausführungsbeispiel in einem Haltearm 1 aufgenommen, wie das bereits mit Bezug auf Figur 1 grundsätzlich beschrieben wurde. Die schematische Darstellung der Manipulatorvorrichtung 100 ist identisch zu der schematischen Darstellung der Manipulatorvorrichtung 100 aus Figur 24 und insofern wird vollumfänglich auf die obige Beschreibung Bezug genommen. Gleiche und ähnliche Elemente sind zudem mit gleichen Bezugszeichen versehen.

Zunächst ist der Haltearm 1 fest an einem Operationstische befestigt und hat ein Koordinatensystem KS0, das ein Basiskoordinatensystem ist. In dem Koordinatensystem KS0 befindet sich auch der Patient, da sich die Position des Patienten in der Regel während einer Operation relativ zur Basis 10 des Haltearms 1 nicht bewegt.

Über die beschriebene Pivotpunktlehre 320 kann ein Tool Center Point Koordinatensystem KS_{TCP} bestimmt werden, das im ersten Pivotpunkt PT1 liegt. Der Pivotpunkt PT1 ist derjenige Pivotpunkt, der mittels der Pivotpunktlehre 320 über den Tastkopf 324 festgelegt wurde. Die Z-Achse des Koordinatensystems KS_{TCP} zeigt in Richtung der Achse K4, die durch die Instrumentenaufnahmeeinrichtung 120 definiert ist. Die Lage der Achse K4 ist parallel zur Achse L1 des Linearantriebs und kann schräg (wie in Fig. 22) zu den ersten und zweiten Bewegungsebenen B1, B2 verlaufen. Der Anstellwinkel *γ* zwischen der Achse K4 und der ersten Bewegungsebene B1 wird durch eine unterschiedliche Betätigung der ersten und zweiten Lenkeranordnungen 110, 112 definiert.

Wenn nun ein anderes chirurgisches Instrument 112 aufgenommen wird, liegt der Pivotpunkt PT1 zunächst an der Stelle, die mittels der Pivotpunktlehre 320 eingelesen wurde. Der Pivotpunkt PT1 kann aber auch durch Berechnung entlang der Längsachse K4 verschoben werden. So verschiebt sich beispielsweise der aktuelle Pivotpunkt vom Pivotpunkt PT1 aus hin zum Pivotpunkt PT2, wenn das chirurgische Instrument 102 in den Körper eines Patienten eingeführt wird. So kann beispielsweise das Endoskop, welches bei fortschreitender Operation auch fortschreitend mitgeführt werden soll, um den OP-Bereich wiederzugeben, bei einer sogenannten Schlüsselloch-OP immer so verschoben werden, dass der Pivotpunkt etwa im Bereich des Schlüssellochs liegt. In Einzelfällen kann der Pivotpunkt auch außerhalb der Achse K4 liegen.

Vorzugsweise wird zudem eine Transformationsmatrix zwischen der Basis 10, das heißt dem Basiskoordinatensystem KS0, und einem der Koordinatensysteme am Pivotpunkt KS_{PVP}, oder dem initialen Pivotpunkt KS_{TCP} ermittelt. Vorzugsweise wird auch diese Transformationsmatrix über die Schnittstelle 106 bereitgestellt und/oder in dem Speicher 282 abgespeichert.

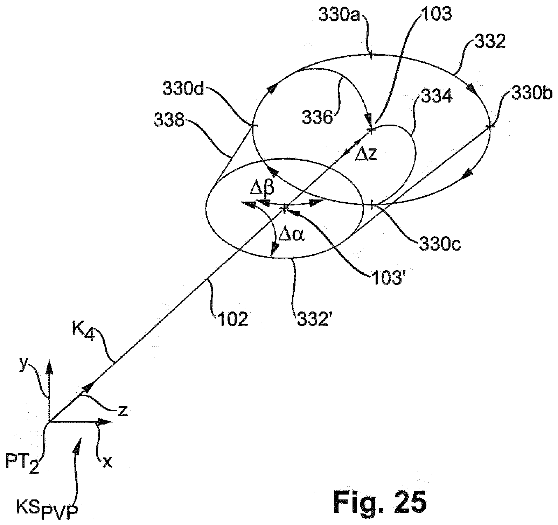

Die Definition des Pivotpunkts PT1, PT2 und dessen Abspeicherung lässt sich auch bei einem zyklischen des chirurgischen Instruments 102 nutzen. Ein solches zyklisches Schwenken wird beispielsweise von Bedienern genutzt, um einen räumlichen Eindruck des durch ein Endoskop beobachteten Feldes zu gewinnen. Für den Fall, dass als chirurgisches Instrument 102 ein Endoskop aufgenommen ist, ist das folgende Verfahren bevorzugt, welches mit Bezug auf Figur 25 genauer erläutert werden wird: (1.) Der Bediener positioniert das Endoskop; (2.) der Bediener startet Pivotierbewegung bzw. automatischer Start durch die Steuereinheit 280 nach Positionierung; (3.) Ermitteln von Stützpunkten 330a, 330b, 330c, 330d relativ zur aktuellen Pose für eine Endoskopspitze 103, wobei die Stützpunkte 330a, 330b, 330c, 330d gemeinsam eine Bahn 332 definieren. Die Bahn 332 kann dabei geplant werden, d.h. stetig durch die Stützpunkt 330a, 330b, 330c, 330d verlaufen. Alternativ werden die Stützpunkte 330a, 330b, 330c, 330d direkt und diskret angefahren. Vorzugsweise ist die Bahn 332 elliptisch. Die elliptische Bahn 332 wird vorzugsweise von der Steuereinheit 280 unter Verwendung der Stützpunkte 330a, 330b, 330c, 330d ermittelt. Anschließend wird das Instrument 102 auf die Bahn 332 eingeschwenkt. Hierzu ist eine Einschwenkbahn 334 erforderlich. Das Instrument 102 wird dann vorzugsweise so bewegt, dass die Spitze 103 entlang der Bahn 332 verfährt, bis ein entsprechendes Abbruchsignal an der Steuereinheit 280 empfangen, oder von dieser erzeugt wird. Zum Ausschwenken zurück zur Ausgangslage ist dann eine Ausschwenkbahn 336 vorgesehen.

Wenn das Instrument 102 entlang seiner Längsachse K4 in Z-Richtung bezogen auf das Pivotpunkt-Koordinatensystem KS_{PVP} verschoben wird, wird kommt die Spitze 103 beispielsweise zu einem Punkt, der in Figur 25 mit 103' bezeichnet ist; das Instrument 102 ist also ein Stück ΔZ verschoben. Für diesen Fall wird die Bahn 332 zur Bahn 332' transformiert, die auf dem Hüllkegel 338 liegt, der zwischen dem Pivotpunkt PT2 und der Bahn 332 aufgespannt wird. Das Ermitteln der transformierten Bahn 332' wird vorzugsweise von der Steuereinheit 280 unter Verwendung geeigneter Softwaremittel ausgeführt.

Die chirurgische Manipulatorvorrichtung 100 weist weiterhin bevorzugt eine Anzeigeeinrichtung 350 zum Anzeigen von einem oder mehreren Zuständen der chirurgischen Manipulatorvorrichtung 100 auf. Die allgemeine Funktion einer solche Anzeigeeinrichtung ist auch in EP 3 130 305 A1 mit Bezug auf Gelenke des dort offenbarten und beanspruchten Haltearms beschrieben und diese Lehre kann analog auf die vorliegende Manipulatorvorrichtung, insbesondere deren Gelenke, angewendet werden.

Die Anzeigeeinrichtung 350 kann grundsätzlich in beliebiger Weise ausgebildet sein, beispielsweise ein Display umfassen, oder in dem in den Figuren gezeigten Ausführungsbeispiel umfasst die Anzeigeeinrichtung mehrere Anzeigesegmente, nämlich zunächst ein oberes Anzeigesegment 352 (Fig. 23) und ein unteres Anzeigesegment (Fig. 5). Die Begriffe "oben" und "unten" beziehen sich in diesem Ausführungsbeispiel auf eine Initialstellung der chirurgischen Manipulatorvorrichtung 100, bei der die ersten und zweiten Bewegungsebenen B1, B2 im Wesentlichen horizontal ausgerichtet sind, und das obere Anzeigesegment 352 nach oben zeigt.

Das obere und das untere Anzeigesegment 352, 354 sind als ringförmige Leuchtstreifen ausgebildet, und insbesondere aus einer Mehrzahl an LED-Elementen gebildet. Die beiden Anzeigesegmente 352, 354 sind identisch und spiegelsymmetrisch bezogen auf die erste, bzw. zweite Bewegungsebene B1, B2, ausgebildet. Dies hat den Vorteil, dass bei jeder Lage der chirurgischen Manipulatorvorrichtung 100 ein Bediener entweder das obere Anzeigesegment 352 oder das untere Anzeigesegment 354 sehen kann.

Darüber hinaus verfügt gemäß diesem Ausführungsbeispiel jedes der oberen und unteren Anzeigesegmente 352, 354 über vier Einzelsegmente 355, 356, 357, 358. Dabei ist das erste Anzeigesegment 355 dem ersten Hebelgelenkpunkt 121 zugeordnet, das zweite Anzeigesegment 356 dem zweiten Hebelgelenkpunkt 122 zugeordnet, das dritte Anzeigesegment 357 dem dritten Hebelgelenkpunkt 123 zugeordnet und das vierte Anzeigesegment 358 dem vierten Hebelgelenkpunkt 124 zugeordnet. So ist beispielsweise vorgesehen, dass bei Bewegung eines der Hebelgelenkpunkte 121, 122, 123, 124 beziehungsweise der entsprechenden Hebel 131, 132, 133, 134, das entsprechend zugeordnete Segment 355, 356, 357, 358 beleuchtet wird, um die Betätigung anzuzeigen. Da jeweils zwei der Hebel zusammengeschaltet sind und sich nicht unabhängig voneinander bewegen können, kann auch vorgesehen sein, dass das erste Segment 355 dem ersten Motor zugeordnet ist, das vierte Anzeigesegment 352 dem zweiten Motor zugeordnet ist, das zweite Anzeigesegment 356 dem dritten Motor zugeordnet ist und das dritte Anzeigesegment 358 dem vierten Motor zugeordnet ist, wenn ein Antrieb 210 für die Manipulatorvorrichtung 100 vorgesehen ist. Auf diese Weise findet eine räumliche Zuordnung der vier Anzeigesegmente 355, 356, 357, 358 zu den entsprechenden Motoren 211, 212, 213, 214 statt, die betätigt werden. Auf diese Weise kann der Bediener erkennen, welcher der Motoren 211, 212, 213, 214 betätigt wird, und in welche Richtung sich das Instrument bzw. die ersten und zweiten Halter 114, 116 bzw. das daran aufgenommene chirurgische Instrument 102 bewegen wird.

Ebenso kann vorgesehen sein, dass zu Assistenzzwecken ein Leuchtpunkt entlang der Bahn der ringförmigen oberen bzw. der unteren Anzeigesegmente läuft, um eine Bewegungsrichtung eines Hebels 131 bis 139 anzuzeigen. Dies ist besonders bevorzugt, wenn die Manipulatorvorrichtung 100 als passive Manipulatorvorrichtung 100 ausgebildet ist und eine Bremseinrichtung 220 aufweist. Sind die Bremsen 221, 222, 223, 224 gelöst, kann auf diese Weise mit dem umlaufenden bzw. sich entlang eines Anzeigesegments 355, 356, 357, 358 bewegenden Leuchtpunkts eine Richtung angezeigt werden, in die ein Bediener das chirurgische Instrument 102 bewegen muss, um dies beispielsweise zu einem Pivotpunkt oder in eine andere vorbestimmte Pose zu bringen.

Weitere Möglichkeiten der Anzeige sind oben bereits beschrieben worden. Als Anzeige wird in diesem Sinne insbesondere verstanden, das Versetzen der Segmente 352, 354, teilweise oder vollständig in einen beleuchteten Zustand von einem nicht beleuchteten Zustand; das Wechseln einer Farbe, das Wechseln einer Intensität, das Wechseln einer Blinkfrequenz, oder Intensitätsschwankungsfrequenz, das Anzeigen von einem oder mehreren teilweise umlaufenden Leuchtpunkten mit einer höheren, geringeren Intensität oder einer anderen Farbe.

In einer weiteren Ausführungsform kann auch vorgesehen sein, dass die Anzeigeeinrichtung 350, eine oder mehrere Infrarotleuchtquellen aufweist, über die die Anzeigeeinrichtung 350 mit einem OP-Navigationssystem kommunizieren kann. Die Infrarotleuchte ist vorzugsweise gleichgeschaltet mit den restlichen LEDs und zeigt somit denselben Zustand an, wie die Anzeigeeinrichtung 350 insgesamt. Das heißt, über die Infrarotleuchte kann einem OP-Navigationssystem mitgeteilt werden, dass beispielsweise ein Motor aktiviert wurde, oder ein sonstiger Zustand der Manipulatorvorrichtung 100 geändert wurde.

Es kann auch vorgesehen sein, dass diese Infrarotleuchte dazu eingesetzt wird, andere Daten, wie insbesondere Pivotpunktkoordinaten oder dergleichen, drahtlos an das OP-Navigationssystem zu übertragen.

Auch wenn in diesem Ausführungsbeispiel (Fig. 25) gezeigt ist, dass die Segmente 355, 356, 357, 358 gemeinsam einen Ring bilden, kann in anderen Ausführungsbeispielen auch vorgesehen sein, dass jedes dieser Segmente 355, 356, 357, 358 in sich ringförmig ist und einem Hebelgelenkpunkt 121 bis 129 zugeordnet ist. Es kann auch vorgesehen sein, dass der Ring des oberen und unteren Anzeigesegments 352, 354 nicht geschlossen ist, oder eine andere Geometrie aufweist. Ebenso ist es denkbar, weitere Anzeigeeinrichtungen oder alternative Anzeigeeinrichtungen an den Seiten des Gehäuses 104 vorzusehen.

In Figur 25 ist weiterhin die Schnittstelle 106 gezeigt. Diese weist im Zentrum eine Ausnehmung 360 auf, die eine Mehrzahl an Flanken hat, um formschlüssig mit einem Vorsprung eines Stativs, Haltearms, oder dergleichen zu koppeln. Zur Verriegelung der formschlüssigen Verbindung ist ein Zapfen 362 vorgesehen, der in eine entsprechende Ausnehmung an dem Vorsprung des Haltearms oder Stativs eingreift und so die formschlüssige Verbindung arretiert. Dieser Zapfen kann mit Bezug auf 25 nach oben bewegt werden, indem ein Taster 364 gedrückt wird. Der Taster 365 ist mittels einer Feder (vgl. Fig. 16, 17) in die arretierte Position vorgespannt.

Weiterhin weist die Schnittstelle 106 eine Mehrzahl elektrischer Kontakt auf, die in diesem Ausführungsbeispiel insgesamt mit 366 bezeichnet sind. Ein Kragen 368 springt axial die Schnittstelle 106 umschließend etwas vor, und dient insbesondere zur Abdichtung der Schnittstelle gegenüber der Umgebung. So wird verhindert, dass die elektrischen Kontakte 366 mit Flüssigkeit oder dergleichen in Kontakt kommen.

## Patentansprüche

1. Chirurgische Manipulatorvorrichtung (100) zum Positionieren eines chirurgischen Instruments (102), insbesondere Endoskops, mit
einem Rahmen (108),
einem ersten Halter (114) und einem zweiten Halter (116) zum Halten des das chirurgischen Instruments (102), insbesondere mittels einer Instrumentenaufnahme (120) für das chirurgische Instrument (102),
einer ersten an dem Rahmen (108) gelagerten Lenkeranordnung (110), welche den Rahmen (108) mit dem ersten Halter (114) gelenkig verbindet, und
einer zweiten an dem Rahmen (108) gelagerten Lenkeranordnung (112), welche den Rahmen (108) mit dem zweiten Halter (116) gelenkig verbindet,
wobei die ersten und zweiten Lenkanordnungen (110, 112) jeweils in zueinander parallelen und beabstandeten ersten und zweiten Bewegungsebenen (B1, B2) relativ zum Rahmen (108) bewegbar sind, sodass der erste Halter (114) in der ersten Bewegungsebene (B1) und der zweite Halter (116) in der zweiten Bewegungsebene (B2) bewegbar ist,
wobei die erste Lenkeranordnung (110) an vier Hebelgelenkpunkten (121, 122, 123, 124) der ersten Lenkeranordnung (110) mit dem Rahmen (108) gekoppelt ist, und die zweite Lenkeranordnung (112) an vier Hebelgelenkpunkten (125, 126, 127, 128) der zweiten Lenkeranordnung (112) mit dem Rahmen (108) gekoppelt ist,
wobei jeweils ein Hebelgelenkpunkt (121, 122, 123, 124) der ersten Lenkeranordnung (110) eine gemeinsame Rotationsachse (R1, R2, R3, R4) mit einem Hebelgelenkpunkt (125, 126, 127, 128) der zweiten Lenkeranordung (112) hat.

2. Chirurgische Manipulatorvorrichtung nach Anspruch 1, wobei die vier ersten Hebelgelenkpunkte (121, 122, 123, 124) und die vier zweiten Hebelgelenkpunkte (125, 126, 127, 128) jeweils V-förmig angeordnet sind.

3. Chirurgische Manipulatorvorrichtung nach einem der vorstehenden Ansprüche, wobei die vier Hebelgelenkpunkte (121, 122, 123, 124) der ersten Lenkeranordnung (110) und die vier Hebelgelenkpunkte (125, 126, 127, 128) der zweiten Lenkeranordnung (112) auf gemeinsamen vier Rotationsachsen (R1, R2, R3, R4) angeordnet sind, oder die vier Hebelgelenkpunkte (121, 122, 123, 124) der ersten Lenkeranordnung (110) und die vier Hebelgelenkpunkte (125, 126, 127, 128) der zweiten Lenkeranordnung (112) parallel versetzt zueinander sind.

4. Chirurgische Manipulatorvorrichtung nach einem der vorstehenden Ansprüche, wobei die erste Lenkeranordnung (110) aufweist:
einen ersten (131), einen zweiten (132), einen dritten (133) und einen vierten Hebel (134), die jeweils drehbar an ersten, zweiten, dritten und vierten Hebelgelenkpunkten (121, 122, 123, 124) der ersten Lenkeranordnung (110) an dem Rahmen (108) drehbar gelagert sind;
einen ersten Lenker (141), der mit den ersten und zweiten Hebeln (131, 132) drehbar gekoppelt ist und einen zweiten Lenker (142), der mit den dritten und vierten Hebeln (133, 134) drehbar gekoppelt ist; und
erste und zweite Stäbe (151, 152), die einerseits drehbar mit dem ersten Lenker (141) und andererseits drehbar mit dem ersten Halter (114) gekoppelt sind, sowie dritte und vierte Stäbe (153, 154), die einerseits drehbar mit dem zweiten Lenker (142) und andererseits drehbar mit dem ersten Halter (114) gekoppelt sind.

5. Chirurgische Manipulatorvorrichtung nach einem der vorstehenden Ansprüche, wobei die erste Lenkeranordnung (119) ein erstes (91), ein zweites (92), ein drittes (93) und ein viertes Parallelogramm (94) aufweist, wobei vorzugsweise das erste und zweite Parallelogramm (91, 92) einen gemeinsamen Gelenkpunkt (161) aufweisen, und/oder das dritte und vierte Parallelogramm (93, 94) einen gemeinsamen Gelenkpunkt (166) aufweisen.

6. Chirurgische Manipulatorvorrichtung nach einem der vorstehenden Ansprüche, aufweisend einen Antrieb (210) für die ersten und zweiten Lenkeranordnungen (110, 112), wobei der Antrieb (210) einen ersten und einen zweiten Motor (211, 212) für die erste Lenkeranordnung (110), und einen dritten und einen vierten Motor (213, 214) für die zweite Lenkeranordnung (112) aufweist.

7. Chirurgische Manipulatorvorrichtung nach einem der vorstehenden Ansprüche, aufweisend eine Bremseinrichtung (220) zum aktiven Bremsen der ersten und zweiten Lenkeranordnungen (110, 112) sowie eine Freigabeeinheit (225) zum selektiven Freigeben eines oder mehrerer Freiheitsgrade der ersten und/oder zweiten Lenkeranordnung (110, 112), wobei die Bremseinrichtung (220) eine erste und eine zweite Bremse (221, 222) für die erste Lenkeranordnung (110), und eine dritte und eine vierte Bremse (223, 224) für die zweite Lenkeranordnung (112) aufweist.

8. Chirurgische Manipulatorvorrichtung nach einem der vorstehenden Ansprüche, aufweisend eine Instrumentenaufnahmeeinrichtung (120), die gelenkig mit dem ersten und dem zweiten Halter (114, 116) gekoppelt ist, wobei die Instrumentenaufnahmeeinrichtung (120) Formschlussmittel (258) aufweist, die dazu eingerichtet sind, ein Ankopplungselement (262) für das chirurgische Instrument (102) aufzunehmen, und wobei das Ankopplungselement (262) aus einem Isolationsmaterial gebildet ist, um falls ein chirurgisches Instrument (102) in dem Ankopplungselement (262) aufgenommen ist, dieses gegenüber der Instrumentenaufnahmeeinrichtung (120) und den ersten und zweiten Haltern (114, 116) elektrisch zu isolieren.

9. Chirurgische Manipulatorvorrichtung nach Anspruch 8, wobei die Instrumentenaufnahmeeinrichtung (120) einen Linearantrieb (230) aufweist, zum Positionieren des chirurgischen Instruments (102) wenigstens teilweise Senkrecht zu den ersten und zweiten Bewegungsebenen (B1, B2).

10. Chirurgische Manipulatorvorrichtung nach einem der vorstehenden Ansprüche, aufweisend eine elektronische Schnittstelle (106) zum Empfangen von Stellsignalen von einer übergeordneten Steuereinheit (290), insbesondere eines OP-Navigationssystems oder einem chirurgischen Haltearm (1) mit der übergeordneten Steuereinheit (290).

11. Chirurgische Manipulatorvorrichtung nach einem der vorstehenden Ansprüche, aufweisend ein Gehäuse (104) mit einer Anzeigeeinrichtung (350) zum Anzeigen von einem oder mehreren Zuständen der chirurgischen Manipulatorvorrichtung (100), wobei vorzugsweise die Anzeigeeinrichtung (350) zwei, vorzugsweise vier, Anzeigesegmente aufweist, wobei jedes Anzeigesegment zwei oder einem der vier Hebelgelenkpunkte der ersten und/oder zweiten Lenkeranordnung (110, 112) zugeordnet ist.

## Claims

1. A surgical manipulator device (100) for positioning a surgical instrument (102), in particular an endoscope, with
a frame (108),
a first mount (114) and a second mount (116) configured to mount the surgical instrument (102), in particular by means of an instrument receptacle (120) for the surgical instrument (102),
a first suspension arm arrangement (110) mounted on the frame (108), to connect the first frame (108) to the first mount (114) in an articulated manner, and
a second suspension arm arrangement (112) supported on the frame (108), and configured to connect the frame (108) to the second mount (116) in an articulated manner,
wherein the first and the second suspension arm arrangements (110, 112) are spaced apart and each displaceable relative to the frame (108) in first and second motion planes (B1, B2) parallel to each other, such that the first mount (114) is displaceable in the first motion plane (B1) and the second mount (116) is displaceable in the second motion plane (B2),
wherein the first suspension arm arrangement (110) is coupled to the frame (108) at four first lever pivot points (121, 122, 123, 124) of the first suspension arm arrangement (110) with the frame (108), and the second suspension arm arrangement (112) is coupled to the frame (108) at four lever pivot points (125, 126, 127, 128) of the second suspension arm arrangement (112) to the frame (108),
wherein each respective lever pivot point (121, 122, 123, 124) of the first suspension arm arrangement (110) has a common axis of rotation (R1, R2, R3, R4) with a respective lever pivot point (125, 126, 127, 128) of the second suspension arm arrangement (112).

2. The surgical manipulator device according to claim 1, wherein the four first lever pivot points (121, 122, 123, 124) and the four second lever pivot points (125, 126, 127, 128) are each arranged in a V-shape.

3. The surgical manipulator device according to one of the preceding claims, wherein the four lever pivot points (121, 122, 123, 124) of the first suspension arm arrangement (110) and the four lever pivot points (125, 126, 127, 128) of the second suspension arm arrangement (112) are arranged on four common axes of rotation (R1, R2, R3, R4), or the four lever pivot points (121, 122, 123, 124) of the first suspension arm arrangement (110) and the four lever pivot points (125, 126, 127, 128) of the second suspension arm arrangement (112) are offset parallel to each other.

4. The surgical manipulator device according to one of the preceding claims, wherein the first suspension arm assembly (110) comprises:
a first (131), a second (132), a third (133), and a fourth lever (134), each of which is rotatably mounted at first, second, third, and fourth lever pivot points (121, 122, 123, 124) of the first suspension arm assembly (110) on the frame (108);
a first suspension arm (141) pivotally coupled to the first and second levers (131, 132) and a second suspension arm (142) pivotally coupled to the third and fourth levers (133, 134); and
first and second rods (151, 152) that are rotatably coupled on one side to the first suspension arm (141) and on the other side to the first mount (114), as well as third and fourth rods (153, 154) that are rotatably coupled on one side to the second suspension arm (142) on one side and rotatably coupled to the first mount (114) on the other side.

5. The surgical manipulator device according to one of the preceding claims, wherein the first suspension arm arrangement (119) comprises a first (91), a second (92), a third (93), and a fourth parallelogram (94), wherein preferably the first and second parallelograms (91, 92) comprise a common pivot point (161), and/or the third and fourth parallelograms (93, 94) comprise a common pivot point (166).

6. The surgical manipulator device according to one of the preceding claims, comprising a drive (210) for the first and second suspension arm assemblies (110, 112), wherein the drive (210) comprises a first and a second motor (211, 212) for the first suspension arm assembly (110), and a third and fourth motor (213, 214) for the second suspension arm assembly (112).

7. The surgical manipulator device according to one of the preceding claims, comprising a braking device configured to (220) actively brake the first and second suspension arm arrangements (110, 112) and a releasing unit (225) configured to selectively release one or more degrees of freedom of the first and/or second suspension arm arrangments (110, 112), wherein the braking device (220) comprises a first and a second brake for the first suspension arm arrangement (221, 222) (110), and a third and a fourth brake (223, 224) for the second suspension arm arrangement (112).

8. The surgical manipulator device according to one of the preceding claims, comprising an instrument receiving device (120) coupled to the first and the second mount (114, 116) in an articulated manner, wherein the instrument receiving device (120) comprises form-fit means (258) set up for receiving a coupling element (262) for the surgical instrument (102), and wherein the coupling element (262) is formed from an insulating material in order to electrically insulate a surgical instrument (102), if received in the coupling element (262), relative to the instrument receiving device (120) and the first and second mounts (114, 116).

9. The surgical manipulator device according to claim 8, wherein the instrument receiving device (120) comprises a linear drive (230) for positioning the surgical instrument (102) at least partially perpendicular to the first and second motion planes (B1, B2).

10. The surgical manipulator device according to one of the preceding claims, comprising an electronic interface (106) for receiving control signals from a higher-level control unit (290), in particular an operating room navigation system or a surgical holding arm (1) with the higher-level control unit (290).

11. The surgical manipulator device according to one of the preceding claims, comprising a housing (104) with a display device (350) configured to display one or more states of the surgical manipulator device (100), wherein the display device (350) preferably has two, preferably four, display segments, each display segment being assigned to two or one of the four lever pivot points of the first and/or second suspension arm arrangement (110, 112).

## Revendications

1. Dispositif manipulateur chirurgical (100) destiné à positionner un instrument chirurgical (102), en particulier un endoscope, comportant
un cadre (108),
un premier élément de maintien (114) et un deuxième élément de maintien (116) destinés à maintenir l'instrument chirurgical (102), en particulier au moyen d'un logement d'instrument (120) pour l'instrument chirurgical (102),
un premier arrangement de biellette (110) monté sur le cadre (108), qui relie de manière articulée le cadre (108) au premier élément de maintien (114), et
un deuxième arrangement de biellette (112) monté sur le cadre (108), qui relie de manière articulée le cadre (108) au deuxième élément de maintien (116),
les premier et deuxième arrangements de biellette (110, 112) étant chacun mobiles par rapport au cadre (108) dans des premier et deuxième plans de déplacement (B1, B2) parallèles l'un à l'autre et espacés l'un de l'autre, de sorte que le premier élément de maintien (114) est mobile dans le premier plan de déplacement (B1) et le deuxième élément de maintien (116) est mobile dans le deuxième plan de déplacement (B2),
le premier arrangement de biellette (110) étant couplé au cadre (108) en quatre points d'articulation de levier (121, 122, 123, 124) du premier arrangement de biellette (110), et le deuxième arrangement de biellette (112) étant couplé au cadre (108) en quatre points d'articulation de levier (125, 126, 127, 128) du deuxième arrangement de biellette (112),
un point d'articulation de levier (121, 122, 123, 124) du premier arrangement de biellette (110) ayant respectivement un axe de rotation commun (R1, R2, R3, R4) avec un point d'articulation de levier (125, 126, 127, 128) du deuxième arrangement de biellette (112).

2. Dispositif manipulateur chirurgical selon la revendication 1, les quatre premiers points d'articulation de levier (121, 122, 123, 124) et les quatre deuxièmes points d'articulation de levier (125, 126, 127, 128) étant chacun disposés en forme de V.

3. Dispositif manipulateur chirurgical selon l'une quelconque des revendications précédentes, les quatre points d'articulation de levier (121, 122, 123, 124) du premier arrangement de biellette (110) et les quatre points d'articulation de levier (125, 126, 127, 128) du deuxième arrangement de biellette (112) étant situés sur quatre axes de rotation communs (R1, R2, R3, R4), ou les quatre points d'articulation de levier (121, 122, 123, 124) du premier arrangement de biellette (110) et les quatre points d'articulation de levier (125, 126, 127, 128) du deuxième arrangement de biellette (112) étant décalés parallèlement les uns par rapport aux autres.

4. Dispositif manipulateur chirurgical selon l'une quelconque des revendications précédentes, le premier arrangement de biellette (110) comprenant:
un premier (131), un deuxième (132), un troisième (133) et un quatrième levier (134), qui sont montés respectivement de manière rotative sur le cadre (108) au niveau de premiers, deuxièmes, troisièmes et quatrièmes points d'articulation de levier (121, 122, 123, 124) du premier arrangement de biellette (110);
une première biellette (141) qui est couplée de manière rotative aux premier et deuxième leviers (131, 132) et une deuxième biellette (142) qui est couplée de manière rotative aux troisième et quatrième leviers (133, 134); et
des première et deuxième barres (151, 152) qui sont d'une part couplées de manière rotative à la première biellette (141) et d'autre part couplées de manière rotative au premier élément de maintien (114), ainsi que des troisième et quatrième barres (153, 154) qui sont d'une part couplées de manière rotative à la deuxième biellette (142) et d'autre part couplées de manière rotative au premier élément de maintien (114).

5. Dispositif manipulateur chirurgical selon l'une quelconque des revendications précédentes, le premier arrangement de biellette (119) présentant un premier (91), un deuxième (92), un troisième (93) et un quatrième parallélogramme (94), de préférence, le premier et le deuxième parallélogramme (91, 92) ayant un point d'articulation (161) commun et/ou le troisième et le quatrième parallélogramme (93, 94) ayant un point d'articulation (166) commun.

6. Dispositif manipulateur chirurgical selon l'une quelconque des revendications précédentes, présentant un entraînement (210) pour les premier et deuxième arrangements de biellette (110, 112), l'entraînement (210) présentant un premier et un deuxième moteur (211, 212) pour le premier arrangement de biellette (110), et un troisième et un quatrième moteur (213, 214) pour le deuxième arrangement de biellette (112).

7. Dispositif manipulateur chirurgical selon l'une quelconque des revendications précédentes, présentant un dispositif de freinage (220) destiné à freiner activement les premier et deuxième arrangements de biellette (110, 112) ainsi qu'une unité de libération (225) destinée à libérer sélectivement un ou plusieurs degrés de liberté des premier et/ou deuxième arrangements de biellette (110, 112), le dispositif de frein (220) présentant un premier et un deuxième frein (221, 222) pour le premier arrangement de biellette (110), et un troisième et un quatrième frein (223, 224) pour le deuxième arrangement de biellette (112).

8. Dispositif manipulateur chirurgical selon l'une quelconque des revendications précédentes, présentant un dispositif de réception d'instrument (120) qui est couplé de manière articulée au premier et au deuxième élément de maintien (114, 116), le dispositif de réception d'instrument (120) présentant des moyens de verrouillage par complémentarité de formes (258) qui sont adaptés pour recevoir un élément d'accouplement (262) pour l'instrument chirurgical (102), et l'élément d'accouplement (262) étant constitué d'un matériau isolant pour, si un instrument chirurgical (102) est reçu dans l'élément d'accouplement (262), isoler celui-ci électriquement du dispositif de réception d'instrument (120) et des premiers et deuxièmes éléments de maintien (114, 116).

9. Dispositif manipulateur chirurgical selon la revendication 8, le dispositif de réception d'instrument (120) présentant un entraînement linéaire (230) destiné à positionner l'instrument chirurgical (102) au moins partiellement perpendiculairement aux premier et deuxième plans de déplacement (B1, B2).

10. Dispositif manipulateur chirurgical selon l'une quelconque des revendications précédentes, présentant une interface électronique (106) destinée à recevoir des signaux de réglage depuis une unité de commande de niveau supérieur (290), en particulier un système de navigation opératoire ou un bras de maintien chirurgical (1) avec l'unité de commande de niveau supérieur (290).

11. Dispositif manipulateur chirurgical selon l'une quelconque des revendications précédentes, présentant un boîtier (104) comportant un dispositif d'affichage (350) destiné à afficher un ou plusieurs états du dispositif manipulateur chirurgical (100), le dispositif d'affichage (350) présentant de préférence deux, de préférence quatre segments d'affichage, chaque segment d'affichage étant associé à deux ou à l'un des quatre points d'articulation de levier du premier et/ou du deuxième arrangements de biellette (110, 112).
